(19) Europäisches Patentamt
European Patent Office
Office européen des brevets

(11) Publication number: **0 167 349 B1**

# (12) EUROPEAN PATENT SPECIFICATION

(45) Date of publication of patent specification: **30.08.89**

(21) Application number: **85304555.7**

(22) Date of filing: **26.06.85**

(51) Int. Cl.$^4$: **A 01 N 37/02**, C 07 C 69/708, C 07 C 79/35, C 07 C 121/38, C 07 C 103/175, C 07 C 67/31, C 07 C 41/16, C 07 C 41/26, C 07 C 45/64, A 01 N 37/48, A 01 N 37/18

## (54) Meta-substituted dioxa-alkane carboxylic acids, esters, amides and salts of diphenyl ethers.

(30) Priority: **02.07.84 US 626952**

(43) Date of publication of application: **08.01.86 Bulletin 86/02**

(45) Publication of the grant of the patent: **30.08.89 Bulletin 89/35**

(84) Designated Contracting States: **AT BE CH DE FR GB IT LI LU NL SE**

(56) References cited:
EP-A-0 004 317
EP-A-0 022 626
EP-A-0 064 658
EP-A-0 077 963
US-A-4 059 435
US-A-4 419 124

(73) Proprietor: **ROHM AND HAAS COMPANY**
**Independence Mall West**
**Philadelphia Pennsylvania 19105 (US)**

(72) Inventor: **James, Michael Philip**
**1012 Meadowview Lane**
**Mont Clare, Pa. 19453 (US)**
Inventor: **Kilbourn, Edward Essex**
**807 North 29th Street**
**Philadelphia, Pa. 19130 (US)**
Inventor: **Yih, Roy Yangming**
**94 Windover Lane**
**Doylestown, Pa. 18901 (US)**

(74) Representative: **Angell, David Whilton et al**
**ROHM AND HAAS (UK) LTD. European Operations Patent Department Lennig House**
**2 Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 167 349 B1

Courier Press, Leamington Spa, England.

## Description

This invention is concerned with meta-substituted dioxa-alkane carboxylic acids, esters, amides and salts of diphenyl ethers which are novel herbicidal compounds, compositions containing them and methods of controlling weeds using them.

Certain diphenyl ethers have been shown to be effective agents for controlling weeds. The herbicidal effectiveness of a given diphenyl ether, however, cannot be predicted from an examination of the substituent groups attached to the phenyl ring in the ether. Frequently, closely related compounds will demonstrate quite different weed control capabilities. Various diphenyl ethers may overlap or have complementary areas of activity or selectivity, and therefore be useful in combination to control a variety of weeds upon application of a single composition.

Furthermore, previously known diphenyl ethers are not completely effective. An ideal herbicide should provide selective weed control over its full growing season with a single administration at low dosages of application. It should be able to control all common weeds by killing them as the seed, the germinating seed, the seedling, and the growing plant. At the same time, the herbicide should not be phytotoxic to the crops to which it is applied and should decompose or otherwise dissipate so as not to poison the soil permanently. The known diphenyl ether herbicides fall short of these ideals. It would be desirable to have new herbicides which show even more selective control of undesirable plants among desirable crop plants or which complement the known diphenyl ethers in activity.

This invention provides diphenyl ethers which have the formula:

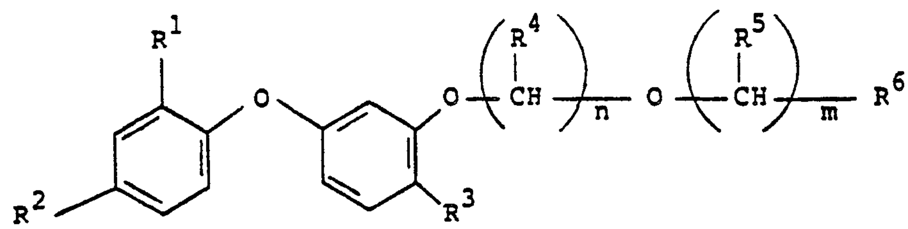

(I)

wherein

$R^1$ is hydrogen, halogen (chloro, fluoro, bromo or iodo), nitro or cyano;

$R^2$ is halogen (chloro, fluoro, bromo or iodo), trifluoromethyl or cyano;

$R^3$ is hydrogen, nitro, halogen (chloro, fluoro, bromo or iodo), cyano, or $(C_1—C_4)$alkylthio, $(C_1—C_4)$alkylsulfinyl or $(C_1—C_4)$alkylsulfonyl;

$R^4$ is hydrogen or $(C_1—C_4)$ straight or branched chain alkyl such as methyl, ethyl, *n*- or *i*-propyl and *n*- or *s*-butyl;

$R^5$ is hydrogen or $(C_1—C_4)$ straight or branched chain alkyl such as methyl, ethyl, *n*- or *i*-propyl and *n*- or *s*-butyl;

$R^6$ is cyano or carboxy or an agronomically acceptable salt, ester or amide thereof;

n is an integer of one to four; and

m is an integer of one to four.

In this specification the term "lower" as applied to an alkyl group means "$(C_1—C_4)$".

We have found the novel compounds of the present invention to show unexpected activity as weed control agents. Preferred embodiments of the invention include those wherein one or more of the following individual preferences apply: $R^1$ is halogen, particularly preferred is chloro; $R^2$ is trifluoromethyl; $R^3$ is nitro or halogen (fluoro, chloro or bromo being particularly preferred); $R^4$ is hydrogen, methyl or ethyl; $R^5$ is hydrogen, methyl or ethyl; $R^6$ is a carbalkoxy group $(CO_2R^7)$ where $R^7$ is a lower $(C_1—C_4)$ straight or branched chain alkyl substituent; n is an integer of one, two or three; and m is one or two.

Examples of the compounds of the invention embraced by Formula I include:

2-chloro-4-trifluoromethyl-3′-(1,4-dioxa-5-carbomethoxy-*n*-hexyl)diphenyl ether
2-chloro-4-trifluoromethyl-3′-(1,4-dioxa-5-carbomethoxy-*n*-hexyl)-4′-nitrodiphenyl ether
2-chloro-4-trifluoromethyl-3′-(1,4-dioxa-5-carbomethoxy-*n*-hexyl)-4′-bromodiphenyl ether
2-chloro-4-trifluoromethyl-3′-(1,4-dioxa-5-carboethoxy-*n*-hexyl)-4′-nitrodiphenyl ether
2-chloro-4-trifluoromethyl-3′-(1,4-dioxa-3-methyl-5-carbomethoxy-*n*-hexyl)-4′-nitrodiphenyl ether
2-chloro-4-trifluoromethyl-3′-(1,4-dioxa-5-carboxy-*n*-hexyl)-4′-nitrodiphenyl ether
2-chloro-4-trifluoromethyl-3′-(1,4-dioxa-3-methyl-5-carbomethoxy-*n*-pentyl)-4′-nitrodiphenyl ether
2-chloro-4-trifluoromethyl-3′-(1,4-dioxa-2-methyl-5-carbomethoxy-*n*-pentyl)-4′-nitrodiphenyl ether
2-chloro-4-trifluoromethyl-3′-(1,3-dioxa-4-carbomethoxy-*n*-pentyl)-4′-nitrodiphenyl ether
2-chloro-4-trifluoromethyl-3′-(1,4-dioxa-5-carbomethoxy-*n*-pentyl)-4′-nitrodiphenyl ether
2-chloro-4-trifluoromethyl-3′-(1,4-dioxa-5-cyano-*n*-hexyl)-4′-nitrodiphenyl ether
2-chloro-4-trifluoromethyl-3′-(1,4-dioxa-5-carboethoxy-*n*-pentyl)-4′-nitrodiphenyl ether
2-chloro-4-trifluoromethyl-3′-(1,5-dioxa-6-carbomethoxy-*n*-hexyl)-4′-nitrodiphenyl ether
2-chloro-4-trifluoromethyl-3′-(1,4-dioxa-5-carboisopropoxy)-*n*-pentyl)-4′-nitrodiphenyl ether
2-chloro-4-trifluoromethyl-3′-(1,5-dioxa-6-carbomethoxy-*n*-heptyl)-4′-nitrodiphenyl ether
2-chloro-4-trifluoromethyl-3′-(1,4-dioxa-5-carbomethoxy-*n*-heptyl)-4′-nitrodiphenyl ether

2-chloro-4-trifluoromethyl-3′-(1,4-dioxa-5-carboisopropoxy-*n*-hexyl-4′-nitrodiphenyl ether
2-chloro-4-trifluoromethyl-3′-(1,4-dioxa-5-carbomethoxy-*n*-pentyl)-4′-bromodiphenyl ether
4-trifluoromethyl-3′-(1,4-dioxa-5-carbomethoxy-*n*-pentyl)-4-nitrodiphenyl ether
2-nitro-4-trifluoromethyl-3′-(1,4-dioxa-carbomethoxy-*n*-pentyl)-4′-nitrodiphenyl ether
2-cyano-4-trifluoromethyl-3′-(1,4-dioxa-5-carbomethoxy-*n*-pentyl)-4-nitrodiphenyl ether
2,4-dichloro-3′-(1,4-dioxa-5-carbomethoxy-*n*-pentyl)-4′-nitrodiphenyl ether
2-chloro-4-cyano-3′-(1,4-dioxa-5-carbomethoxy-*n*-pentyl)-4′-nitrodiphenyl ether
2-chloro-4-trifluoromethyl-3′-(1,4-dioxa-5-carbomethoxy-*n*-pentyl)-4′-fluorodiphenyl ether
2-chloro-4-trifluoromethyl-3′-(1,4-dioxa-5-carbomethoxy-*n*-pentyl)-4′-cyanodiphenyl ether
2-chloro-4-trifluoromethyl-3′-(1,3-dioxa-4-carbomethoxy-*n*-butyl)-4′-nitrodiphenyl ether
2-chloro-4-trifluoromethyl-3′-(1,4-dioxa-2,3-dimethyl-5-carbomethoxy-*n*-pentyl)-4′-nitrodiphenyl ether
2-chloro-4-trifluoromethyl-3′-(1,4-dioxa-5-carbomethoxy-*n*-pentyl)-4′-methylthio diphenyl ether
2-chloro-4-trifluoromethyl-3′-(1,4-dioxa-5-carbomethoxy-*n*-pentyl)-4′-methylsulfinyl diphenyl ether
2-chloro-4-trifluoromethyl-3′-(1,4-dioxa-5-carbomethoxy-*n*-pentyl)-4′-methylsulfonyl diphenyl ether
2-chloro-4-trifluoromethyl-3′-(1,4-dioxa-5-carbomethoxy-*n*-pentyl)diphenyl ether
2-chloro-4-trifluoromethyl-3′-(1,4-dioxa-5-carboxamide-*n*-pentyl)-4′-nitrodiphenyl ether
2-chloro-4-trifluoromethyl-3′-(1,4-dioxa-5-carboxamide-*n*-hexyl)-4′-nitrodiphenyl ether
2-chloro-4-trifluoromethyl-3′-(1,4-dioxa-5-N,N-dimethylcarboxamide-*n*-pentyl)-4′-nitrodiphenyl ether
2-chloro-4-trifluoromethyl-3′-(1,3-dioxa-2-methyl-4-carbomethoxy-*n*-butyl)-4′-nitrodiphenyl ether

When the $R^6$ substituent is carboxamide ($CONR^8R^9$), $R^8$ and $R^9$ are independently a hydrogen atom, straight or branched chain alkyl having up to 4 carbon atoms or straight or branched chain alkoxy having up to 4 carbon atoms.

When the $R^6$ substituent is, or contains, a carboxy group, either the free acid or the salt form can be used. Typical salts are the agronomically acceptable metal salts or ammonium salts. Among the suitable metal salts are those in which the metal cation is an alkali metal cation, such as sodium, potassium or lithium an alkaline earth metal cation, such as calcium, magnesium, barium or strontium, or a heavy metal cation, such as zinc, manganese, cupric, cuprous, ferric, ferrous, titanium or aluminum. Particularly preferred salts include sodium, potassium and ammonium. Among the suitable ammonium salts are those in which the ammonium cation has the formula NWXYZ, wherein each of W, X, Y and Z is, independently, a hydrogen atom, a hydroxy group, a $(C_1—C_4)$alkoxy group, a $(C_1—C_{20})$alkyl group, a $(C_3—C_8)$-alkenyl group, a $(C_3—C_8)$alkynyl group, a $(C_2—C_8)$hydroxyalkyl group, a $(C_2—C_8)$alkoxyalkyl group, a $(C_2—C_6)$aminoalkyl group, a $(C_2—C_6)$haloalkyl group, an amino group, a $(C_1—C_4)$alkyl or di$(C_1—C_4)$alkylamino group, a substituted or unsubstituted phenyl group, a substituted or unsubstituted phenylalkyl group, having up to 4 carbon atoms in the alkyl moiety, or any two of W, X, Y or Z can be taken together to form with the nitrogen atom a 5- or 6-membered heterocyclic ring, optionally having up to one additional hetero oxygen, nitrogen, or sulfur atom in the ring, and preferably saturated, such as a piperidine, morpholino, pyrrolidino, or piperazino ring, or any three of W, X, Y or Z can be taken together to form with the nitrogen atom a 5- or 6-membered aromatic heterocyclic ring, such as a piperazole or pyridine ring. When the ammonium group contains a substituted phenyl or substituted phenylalkyl group, the substituents will generally be selected from halogen atoms, $(C_1—C_8)$alkyl groups, $(C_1—C_4)$alkoxy groups, hydroxy groups, nitro groups, trifluoromethyl groups, cyano groups, amino groups, $(C_1—C_4)$-alkylthio groups, and the like. Such substituted phenyl groups preferably have up to two such substituents. Representative ammonium cations include ammonium, dimethylammonium, 2-ethylhexylammonium, bis(2-hydroxyethyl)-ammonium, tris(2-hydroxyethyl)-ammonium, dicyclohexylammonium, t-octylammonium, 2-hydroxyethylammonium, morpholinium, piperidinium, 2-phenethylammonium, 2-methyl-benzylammonium, n-hexylammonium, triethylammonium, trimethylammonium, tri(n-butyl)-ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium and 4-methyl-benzyltrimethylammonium.

The novel compounds of the invention are useful both as preemergence and as postemergence herbicides. Preemergence herbicides are ordinarily used to treat the soil in which the desired crop is to be planted by application either before seeding, during seeding or, as in most applications, after seeding and before the crop emerges. Postemegence herbicides are those which are applied after the crop plants have emerged and during their growth period.

The novel compounds of the present invention may be advantageously employed as weedkillers in crops of both monocots and dicots and are particularly active against broadleaf weeds postemergence.

Diphenyl ethers of the invention are useful for controlling weeds in rice crops. When used in transplanted rice crops, the ethers can be applied either preemergence or postemergence to the weeds — that is, they can be applied to the growth medium of the transplanted plants either before the weed plants have emerged or while they are in an early stage of growth. The ethers can be applied to the growth medium either before or after the rice has been transplanted to that medium.

The diphenyl ethers of the invention may be applied in any amount which will give the required control of weeds. A preferred rate of application is from about 0.01 to 2.24 kg/ha (0.01 to 2 lb/acre) and especially preferred from 0.056 to 0.56 kg/ha (0.05 to 0.5 pounds per acre) of the active diphenyl ether of the invention.

Under some conditions, the diphenyl ethers of the invention may advantageously be incorporated into the soil or other growth medium prior to planting a crop. This incorporation can be carried out by any convenient means, including by simply mixing with the soil, by applying the diphenyl ether to the surface of the soil and then disking or dragging into the soil to the desired depth, or by employing a liquid carrier to accomplish the necessary penetration and impregnation.

A diphenyl ether of the invention can be applied to the growth medium or to plants to be treated either by itself, or, as is generally done, as a component in a herbicidal composition or formulation which also comprises an agronomically acceptable carrier. By agronomically acceptable carrier we mean for example any substance which can be used to dissolve, disperse or diffuse a herbicidal compound in the composition without impairing the effectiveness of the herbicidal compound and which by itself has no detrimental effect on the soil, equipment, crops, or agronomic environment. Mixtures of the diphenyl ethers of the present invention may also be used in any of these herbicidal formulations. The herbicidal compositions of the invention can be either solid or liquid formulations or solutions. For example, the diphenyl ethers can be formulated as wettable powders, emulsifiable concentrates, dusts, granular formulations, aerosols or flowable emulsion concentrates. In such formulations, the compounds are extended with a liquid or solid carrier and, when desired, suitable surfactants are incorporated.

It is usually desirable, particularly in postemergence applications, to include adjuvants such as wetting agents, spreading agents, dispersing agents, sticking agents, adhesives and the like, in accordance with agricultural practices. Examples of adjuvants which are commonly used in the art can be found in the John W. McCutcheon, Inc. publication "Detergents and Emulsifiers Annual".

The diphenyl ethers of this invention can be dissolved in any appropriate solvent. Examples of solvents which are useful in the practice of this invention include alcohols, ketones, aromatic hydrocarbons, halogenated hydrocarbons, dimethylformamide, dioxane and dimethyl sulfoxide. Mixtures of these solvents can also be used. The concentration of the active ingredient in the solution will usually be from 2% to 98% with a preferred range being 25% to 75%, by weight.

For the preparation of emulsifiable concentrates, the diphenyl ether can be dissolved in organic solvent such as toluene, xylene, methylated naphthalene, corn oil, pine oil, o-dichlorobenzene, isophorone, cyclohexanone and/or methyl oleate, together with an emulsifying agent which permits dispersion in water. Suitable emulsifiers include, for example, the ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth salts or amine salts of alkylbenzenesulfonates and the fatty alcohol sodium sulfates, having surface-active properties can be used as emulsifiers either alone or in conjunction with an ethylene oxide reaction product. Flowable emulsion concentrates are formulated similarly to the emulsifiable concentrates and include, in addition to the above components, water and a stabilizing agent such as a water-soluble cellulose derivative or a water-soluble salt of a polyacrylic acid. The concentration of the active ingredient in emulsifiable concentrates is usually 10% to 60% and in flowable emulsion concentrates, this can be as high as 75%, by weight.

Wettable powders suitable for spraying can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations is usually 20% to 98%, preferably 40% to 75% by weight. A dispersing agent can constitute about 0.5% to about 3% of the composition, and a wetting agent can constitute from about 0.1% to about 5% of the composition, by weight.

Dusts can be prepared by mixing the compounds of the invention with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing 20% to 80% of active ingredient are commonly made and subsequently diluted to 1% to 10%, by weight, use concentration.

Granular formulations can be prepared by impregnating a solid such as granular Fuller's earth, vermiculite, ground corn cobs, seed hulls, including bran or other grain-hulls, or similar material. A solution of one or more of the diphenyl ethers of the present invention in a volatile organic solvent can be sprayed or mixed with the granular solid and the solvent then removed by evaporation. The granular material may be of any suitable size, with a preferred size range of about 1.19 mm to 0.25 mm (16 to 60 mesh). The diphenyl ether will usually comprise about 2% to about 15% by weight of the granular formulation.

The diphenyl ethers of the present invention can also be mixed with fertilizers or fertilizing materials before their application. In one type of solid fertilizing composition in which the diphenyl ethers may be used, particles of a fertilizer or fertilizing ingredients, such as ammonium sulfate, ammonium nitrate, or ammonium phosphate can be coated with one or more of the ethers. The solid diphenyl ether and solid fertilizing material may also be admixed in blending or mixing equipment, or they can be incorporated with fertilizers in granular formulations. Any relative proportion of diphenyl ether and fertilizer can be used which is suitable for the crops and weeds to be treated. The diphenyl ether will commonly be from about 5% to about 25% by weight of the fertilizing composition. These compositions provide fertilizing materials which promote the rapid growth of desired plants, while at the same time control the growth of undesired plants.

The diphenyl ethers of the present invention may be applied as herbicidal sprays by methods commonly employed, such as conventional high-gallonage hydraulic sprays, low gallonage sprays, air-blast spray, aerial sprays, and dusts. If low volume applications are desired, a solution of the compound is usually used. The dilution and rate of application will usually depend upon such factors as the type of equipment employed, method of application, area to be treated and the type and stage of development of the weeds.

For some applications, it may be desired to add one or more other herbicides along with diphenyl ethers of the present invention, thereby providing additional advantages and effectiveness. When mixtures of herbicides are employed, the relative proportions which are used will depend upon the crop to be treated and the degree of selectivity in weed control desired. Examples of other herbicides which can be used with the diphenyl ethers of the present invention include:

Carboxylic Acids and Derivatives

2,3,6-trichlorobenzoic acid and its salts
2,3,5,6-tetrachlorobenzoic acid and its salts
2-methoxy-3,5,6-trichlorobenzoic acid and its salts
2-methoxy-3,6-dichlorobenzoic acid and its salts
2-methyl-3,6-dichlorobenzoic acid and its salts
2,3-dichloro-6-methylbenzoic acid and its salts
2,4-dichlorophenoxyacetic acid and its salts and esters
2,4,5-trichlorophenoxyacetic acid and its salts and esters
2-methyl-4-chlorophenoxyacetic acid and its salts and esters
2-(2,4,5-trichlorophenoxy)propionic acid and its salts and esters
4-(2,4-dichlorophenoxy)butyric acid and its salts and esters
4-(2-methyl-4-chlorophenoxy)butyric acid and its salts and esters
2,3,6-trichlorophenylacetic acid and its salts
3,6-endoxohexahydrophthalic acid
dimethyl 2,3,5,6-tetrachloroterephthalate
trichloroacetic acid and its salts
2,2-dichloropropionic acid and its salts
2,3-dichloroisobutyric acid and its salts

Carbamic Acid Derivatives

ethyl N,N-di(n-propyl)thiolcarbamate
propyl N,N-di(n-propyl)thiolcarbamate
ethyl N-ethyl-N-(n-butyl)thiolcarbamate
propyl N-ethyl-N-(n-butyl)thiolcarbamate
2-chloroallyl N,N-diethyldithiocarbamate
N-methyldithiocarbamic acid salts
ethyl 1-hexamethyleneiminecarbothiolate
isopropyl N-phenylcarbamate
isopropyl N-(m-chlorophenyl)carbamate
4-chloro-2-butynyl N-(m-chlorophenyl)carbamate
methyl N-(3,4-dichlorophenyl)carbamate

Phenols

dinitro-o-(sec-butyl)phenol and its salts
pentachlorophenol and its salts

Substituted Ureas

3-(3,4-dichlorophenyl)-1,1-dimethylurea
3-phenyl-1,1-dimethylurea
3-(3,4-dichlorophenyl)-3-methoxy-1,1-dimethylurea
3-(4-chlorophenyl)-3-methoxy-1,1-dimethylurea
3-(3,4-dichlorophenyl)-1-n-butyl-1-methylurea
3-(3,4-dichlorophenyl)-1-methoxy-1-methylurea
3-(4-chlorophenyl)-1-methoxy-1-methylurea
3-(3,4-dichlorophenyl)-1,1,3-trimethylurea
3-(3,4-dichlorophenyl)-1,1-diethylurea
dichloral urea

Substituted Triazines

2-chloro-4,6-bis(ethylamino)-s-triazine
2-chloro-4-ethylamino-6-isopropylamino-s-triazine
2-chloro-4,6-bis(methoxypropylamino)-s-triazine

2-methoxy-4,6-bis(isopropylamino)-s-triazine
2-chloro-4-ethylamino-6-(3-methoxypropylamino)-s-triazine
2-methylmercapto-4,6-bis(isopropylamino)-s-triazine
2-methylmercapto-4,6-bis(ethylamino)-s-triazine
2-methylmercapto-4-ethylamino-6-isopropylamino-s-triazine
2-chloro-4,6-bis(isopropylamino)-s-triazine
2-methoxy-4,6-bis(ethylamino)-s-triazine
2-methoxy-4-ethylamino-6-isopropylamino-s-triazine
2-methylmercapto-4-(2-methoxyethylamino)-6-isopropylamino-s-triazine

Diphenyl Ether Derivatives

2,4-dichloro-4'-nitrodiphenyl ether
2,4,6-trichloro-4'-nitrodiphenyl ether
2,4-dichloro-6-fluoro-4'-nitrodiphenyl ether
3-methyl-4'-nitrodiphenyl ether
3,5-dimethyl-4'-nitrodiphenyl ether
2,4'-dinitro-4-trifluoromethyldiphenyl ether
2,4-dichloro-3'-methoxy-4'-nitrodiphenyl ether

Anilides

N-(3,4-dichlorophenyl)propionamide
N-(3,4-dichlorophenyl)methacrylamide
N-(3-chloro-4-methylphenyl)-2-methylpentanamide
N-(3,4-dichlorophenyl)trimethylacetamide
N-(3,4-dichlorophenyl)-alpha,alpha-dimethylvaleramide
N-isopropyl-N-phenylchloroacetamide
N-n-butoxymethyl-N-(2,6-diethylphenyl)chloroacetamide
N-n-methoxymethyl-N-(2,6-diethylphenyl)chloroacetamide

Uracils

5-bromo-3-s-butyl-6-methyluracil
5-bromo-3-cyclohexyl-1,6-dimethyluracil
3-cyclohexyl-5,6-trimethyleneuracil
5-bromo-3-isopropyl-6-methyluracil
3-tert-butyl-5-chloro-6-methyluracil

Nitriles

2,6-dichlorobenzonitrile
diphenylacetonitrile
3,5-dibromo-4-hydroxybenzonitrile
3,5-diiodo-4-hydroxybenzonitrile

Other Organic Herbicides

2-chloro-N,N-diallylacetamide
N-(1,1-dimethyl-2-propynyl)-3,5-dichlorobenzamide
maleic hydrazide
3-amino-1,2,4-triazole
monosodium methanearsonate
disodium methanearsonate
N,N-dimethyl-alpha,alpha-diphenylacetamide
N,N-di(n-propyl)-2,6-dinitro-4-trifluoromethylaniline
N,N-di(n-propyl)-2,6-dinitro-4-methylaniline
N,N-di(n-propyl)-2,6-dinitro-4-methylsulfonylaniline
O-(2,4-dichlorophenyl)-O-methyl-isopropylphosphoramidothioate
4-amino-3,5,6-trichloropicolinic acid
2,3-dichloro-1,4-naphthoquinone
di(methoxythiocarbonyl)disulfide
3-isopropyl-1H-2,1,3-benzothiadiazin-(4)3H-one-2,2-dioxide
6,7-dihydrodipyridol[1,2-a:2',1'-c]pyrazidinium salt
1,1'-dimethyl-4,4'-bipyridinium salts
3,4,5,6-tetrahydro-3,5-dimethyl-2-thio-2H-1,3,5-thiadiazine.

The diphenyl ethers of the present invention, or their precursors, are preferably prepared by reacting a suitably substituted diphenyl ether phenol of Formula II

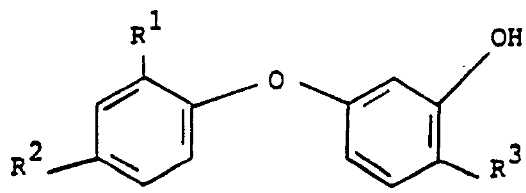

(II)

wherein $R^1$, $R^2$ and $R^3$ are as defined for Formula I, (which are known compounds) in a polar aprotic organic solvent and in the presence of an acid acceptor, with a compound of Formula III

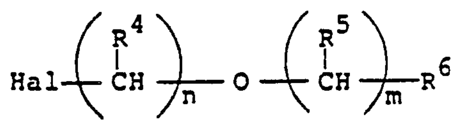

(III)

wherein Hal is halogen, preferably chlorine or bromine and $R^4$, $R^5$, $R^6$, n and m are as defined for Formula I. Examples of suitable bases for the above reaction include KOH, $K_2CO_3$, triethylamine and the like. This reaction is generally carried out at a temperature of 25°C to 200°C, preferably 85°C to 150°C.

Another method for preparing the diphenyl ethers of Formula I is by reacting a suitably substituted diphenyl ether alcohol of Formula IV

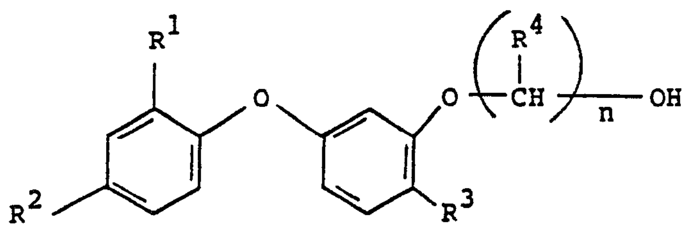

IV

in a polar aprotic organic solvent and in the presence of an acid acceptor with a compound of Formula V

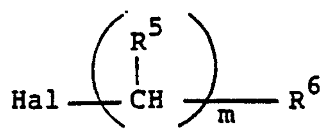

V

wherein Hal is halogen, preferably chlorine or bromine and $R^5$, $R^6$ and m are as defined for Formula I. Examples of suitable bases for the above reaction include NaH and potassium tert-butoxide. This reaction is generally carried out at a temperature of from −30°C to 0°C.

The above reactions are suitably conducted in the presence of polar aprotic organic solvents such as tetrahydrofuran, 1-methyl-2-pyrrolidone, dimethyl sulfoxide, dimethyl formamide, etc. The reaction temperatures are in the range of −30°C to 200°C and the reaction time from about 1 hour to several days, depending on the starting material, reaction temperatures and solvent. The reaction is usually carried out under normal pressure.

Another method for preparing the diphenyl ethers of Formula I is by reacting a suitably substituted diphenyl ether alcohol of Formula IV in a nonprotic organic solvent, such as methylene chloride, and optionally, but preferably, in the presence of a catalyst such as copper with an alkyl diazo compound having the formula VI:

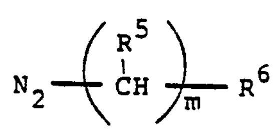

VI

wherein $R^5$, $R^6$ and m are as defined for Formula I.

Diphenyl ethers prepared by the above techniques can also be used as precursors in preparing compounds of the invention. For example, compounds of the invention in which $R^6$ is or includes an ester may be converted by conventional techniques to the corresponding carboxylic acids and amides or alternative carbalkoxy compounds. In addition, compounds of the invention can be made by nitration of suitable precursors without a nitro group prepared by the above techniques.

The compounds of Formula III can be prepared by reacting a suitably substituted alcohol with a suitable aldehyde in the presence of an acid donor, such as hydrochloric acid.

The diphenyl ether alcohol of Formula IV can be prepared by at least two methods, depending upon the desired $R^4$ substituent and n. For example, a suitably substituted diphenyl ether phenol of Formula II is reacted in a polar aprotic organic solvent and in the presence of a suitable acid acceptor such as KOH, $K_2CO_3$, triethylamine and the like with a halo alcohol.

Alternatively, a suitably substituted diphenyl ether phenol of Formula II is reacted in a polar aprotic organic solvent and in the presence of a suitable acid acceptor such as KOH, $K_2CO_3$ or triethylamine with an alpha halocarbonyl derivative and then reduced in a suitable solvent with a suitable reducing agent to obtain the compounds of Formula IV. Examples of solvents include tetrahydrofuran and alcohols such as methanol or ethanol. Examples of reducing agents include borane-dimethyl sulfide complex and sodium borohydride.

The reactions described above for preparing the compounds of Formula IV are generally carried out in polar aprotic organic solvents similar to those previously described.

The salts of the invention can be prepared by any convenient art-recognised method, such as by reacting a metal hydroxide, a metal hydride, or an amine or ammonium salt, such as a halide, hydroxide or alkoxide with the free acid, or reacting a quaternary ammonium salt, such as a chloride, a bromide or nitrate with a metal salt of the invention in a suitable solvent. When metal hydroxides are used as reagents, useful solvents include water, glyme, dioxane, tetrahydrofuran, methanol, ethanol, isopropanol and the like. When metal hydrides are used as reagents, useful solvents include nonhydroxylic solvents such as dioxane, glyme, tetrahydrofuran, diethyl ether, hydrocarbons, including toluene, xylene, hexane, pentane, heptane, and octane, dimethylformamide, and the like. When amines are used as reagents, useful solvents include alcohols, such as methanol or ethanol, hydrocarbons, such as toluene, xylene and hexane, tetrahydrofuran, glyme, dioxane, or water. When ammonium salts are used as reagents, useful solvents include water, alcohols, such as methanol or ethanol, glyme, tetrahydrofuran, or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0°C to about 100°C, preferably at about room temperature.

There should also be mentioned as preferred compounds all of the individual compounds of Formula I hereinbefore and hereinafter disclosed, varied by replacement of one or more of the substituents therein according to one or more of the options given in the list below. Furthermore this list, when one or more of the options in it is applied to the general Formula I, defines further groups of preferred compounds.

The list is as follows:

(a) for $R^1$ : chlorine;
(b) for $R^2$ : trifluoromethyl;
(c) for $R^3$ : bromine, trifluoromethyl, nitro;
(d) for $R^4$ : hydrogen, methyl, ethyl;
(e) for $R^5$ : hydrogen, methyl, ethyl;
(f) for $R^6$ : carbalkoxy ($CO_2R^7$), carboxamide ($CO_2NR^8R^9$);
(g) for $R^7$ : ($C_1$—$C_3$) straight or branched chain alkyl;
(h) for $R^8$ : hydrogen; ($C_1$—$C_4$)-alkyl, ($C_1$—$C_4$)-alkoxy;
(i) for $R^9$ : hydrogen; ($C_1$—$C_4$)-alkyl, ($C_1$—$C_4$)-alkoxy.

The following examples are given for the purposes of illustration only. In Table I, typical diphenyl ethers of the invention are listed with their melting points. These compounds were made by processes of the invention, their structures were confirmed by NMR and in some cases by elemental analysis. Specific details of the preparation of the compounds of Examples 1, 2, 3, 9, 10 and 12 are described after Table I.

**TABLE I**

Diphenyl Ethers - Physical Data

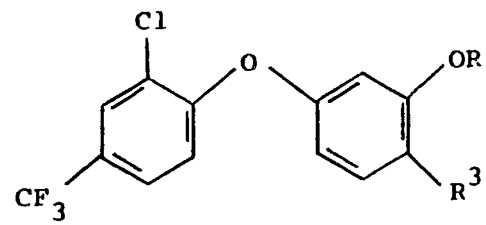

| Example No.* | R | $R^3$ | m.p./b.p. (°C) | | Analysis %C | %H | %N | %Cl | %Br |
|---|---|---|---|---|---|---|---|---|---|
| 1 | $CH_3$<br>$-CH_2CH_2OCHCO_2CH_3$ | H | Oil | Calculated<br>Found | | | | | |
| 2 | $CH_3$<br>$-CH_2CH_2OCHCO_2CH_3$ | $NO_2$ | (217-219°/0.04mm) | Calculated<br>Found | 49.20<br>49.20 | 3.69<br>3.68 | 3.02<br>2.89 | 7.64<br>7.50 | |
| 3 | $CH_3$<br>$-CH_2CH_2OCHCO_2CH_3$ | Br | Oil | Calculated<br>Found | | | | | |
| 4 | $CH_3$<br>$-CH_2CH_2OCHCO_2CH_2CH_3$ | $NO_2$ | Oil | Calculated<br>Found | | | | | |
| 5 | $CH_3CH_3$<br>$-CH_2CHOCHCO_2CH_3$ | $NO_2$ | Oil | Calculated<br>Found | | | | | |

TABLE I - (cont'd)

| Example No.* | R | $R^3$ | m.p./b.p. (°C) | | Analysis %C | %H | %N | %Cl | %Br |
|---|---|---|---|---|---|---|---|---|---|
| 6 | $-CH_2CH_2O\overset{CH_3}{\overset{\vert}{C}}HCO_2H$ | $NO_2$ | Oil | Calculated | | | | | |
| | | | | Found | | | | | |
| 7 | $-CH_2\overset{CH_3}{\overset{\vert}{C}}HOCH_2CO_2CH_3$ | $NO_2$ | 109-110° | Calculated | 49.20 | 3.69 | 3.02 | 7.64 | |
| | | | | Found | 49.10 | 3.80 | 2.98 | 7.53 | |
| 8 | $-\overset{CH_3}{\overset{\vert}{C}}HCH_2OCH_2CO_2CH_3$ | $NO_2$ | Oil | Calculated | | | | | |
| | | | | Found | | | | | |
| 9 | $-CH_2O\overset{CH_3}{\overset{\vert}{C}}HCO_2CH_3$ | $NO_2$ | Oil | Calculated | | | | | |
| | | | | Found | | | | | |
| 10 | $-CH_2CH_2OCH_2CO_2CH_3$ | $NO_2$ | 84-85° | Calculated | 48.07 | 3.36 | 3.11 | 7.88 | |
| | | | | Found | 48.12 | 3.46 | 3.07 | 8.12 | |
| 11 | $-CH_2CH_2O\overset{CH_3}{\overset{\vert}{C}}HC\equiv N$ | $NO_2$ | Oil | Calculated | 50.19 | 3.28 | 6.50 | | |
| | | | | Found | 49.59 | 3.87 | 6.78 | | |
| 12 | $-CH_2CH_2OCH_2CO_2CH_2CH_3$ | $NO_2$ | Oil | Calculated | | | | | |
| | | | | Found | | | | | |

TABLE I - (cont'd)

| Example No.* | R | $R^3$ | m.p./b.p. (°C) | | Analysis %C | %H | %N | %Cl | %Br |
|---|---|---|---|---|---|---|---|---|---|
| 13 | $-CH_2CH_2CH_2OCH_2CO_2CH_3$ | $NO_2$ | Oil | Calculated | | | | | |
| | | | | Found | | | | | |
| 14 | $-CH_2CH_2OCH_2CO_2CH(CH_3)_2$ | $NO_2$ | Oil | Calculated | | | | | |
| | | | | Found | | | | | |
| 15 | $CH_3$<br>$-CH_2CH_2CH_2OCHCO_2CH_3$ | $NO_2$ | Oil | Calculated | | | | | |
| | | | | Found | | | | | |
| 16 | $CH_2CH_3$<br>$-CH_2CH_2OCHCO_2CH_3$ | $NO_2$ | Oil | Calculated | 50.27 | 4.01 | 2.93 | 7.42 | |
| | | | | Found | 51.49 | 4.41 | 3.85 | 7.15 | |
| 17 | $CH_3$<br>$-CH_2CH_2OCHCO_2CH(CH_3)_2$ | $NO_2$ | Oil | Calculated | 51.28 | 4.30 | 2.85 | 7.21 | |
| | | | | Found | 51.58 | 5.06 | 3.85 | 7.21 | |
| 18 | $-CH_2CH_2OCH_2CO_2CH_3$ | Br | Oil | Calculated | 44.70 | 3.12 | - | 7.33 | 16.52 |
| | | | | Found | 44.93 | 3.27 | - | 8.39 | 18.01 |
| 19 | $CH_3$<br>$-CH_2CH_2OCHCON(H)OCH_2CH_3$ | $NO_2$ | Oil | Calculated | 48.73 | 4.09 | 5.68 | 7.19 | |
| | | | | Found | 50.25 | 4.23 | 4.67 | 7.07 | |

TABLE I - (cont'd)

| Example No.* | R | $R^3$ | m.p./b.p. (°C) | | Analysis %C | %H | %N | %Cl | %Br |
|---|---|---|---|---|---|---|---|---|---|
| 20 | $-CH_2CH_2OCH(CH_3)CONH_2$ | $NO_2$ | 116-121 | Calculated | 48.16 | 3.59 | 6.24 | 7.90 | |
| | | | | Found | 48.17 | 3.81 | 5.81 | 8.24 | |

*Note: All compounds were purified by column or high pressure liquid chromatography and structures were confirmed by NMR, IR and, in several instances, by elemental analysis.

Example 1

Preparation of 2-chloro-4-trifluoromethyl-3′-(1,4-dioxa-5-Carbomethoxy-n-hexyl)-diphenyl ether

Sodium hydride (6.92 g of a 50% dispersion in mineral oil; 0.14 mol) was weighed into a liter flask and washed with reagent-grade hexane to remove the mineral oil. Dimethylformamide (300 ml, dried over $CaH_2$) was added and the suspension was cooled to 0° (ice bath). 2-chloro-4-trifluoromethyl-3′-(2-hydroxy-ethoxy)-diphenyl ether (40 g, 0.12 mol) was added dropwise. When addition was complete, the reaction was stirred 5 min. at 0° and then stirred at room temperature until gas evolution stopped. Methyl dl 2-bromopropionate (24.1 g, 0.14 mol) was added rapidly. The reaction was exothermic with gas evolution; stirring was continued for 2½ hours then was heated at 60°C for 1 hr. and finally stirred overnight at room temperature.

The reaction mixture was poured into cold water (500 ml); acidified (5% aqueous HCl) and extracted with MDC (2 × 150 ml). The combined MDC extracts were washed (5% NaOH, $H_2O$, saturated $NaHCO_3$, Brine), dried over anhydrous magnesium sulfate, filtered and the filtrate evaporated under reduced pressure to afford 46.6 g of a light golden oil. The crude material was separated by flash chromatography, using 12—13″ of 60—200 mesh Davison Silica gel in a 40 mm i.d. column/10% ethyl acetate-90% isooctane solvent at a flow rate corresponding to a drop in the fluid level of 2″/min. Twenty ml fractions were collected. Fractions 32—65 contained the product and were combined and evaporated under reduced pressure to give 15.5 g (31%) of a clear, colorless liquid.

The compound of Example 2 was also prepared in a similar manner.

Example 2

Preparation of 2-chloro-4-trifluoromethyl-3′-(1,4-dioxa-5-carbomethoxy-n-hexyl)-4′-nitrodiphenyl ether

*Method A*

Sodium hydride (1.58 g of a 50% dispersion in mineral oil; 0.033 mol) was washed twice with reagent-grade hexane to remove the mineral oil and was suspended in 50 ml of dry DMF. 2-Chloro-4-trifluoromethyl-3′-hydroxy-4′-nitro diphenyl ether (10.01 g; 0.03 mol) was added after the DMF solution was cooled to 0°C. Hydrogen gas evolved and the solution was stirred for 30 minutes at room temperature. After the addition of 2-bromoethanol (11.25 g; 0.09 mol) the reaction was stirred and heated to 80—90°C overnight.

Heating was then discontinued and the solution was diluted by the addition of water (200 ml). The combined MDC extracts were dried over anhydrous magnesium sulfate, filtered and the filtrate concentrated under reduced pressure to afford 11.4 g (100% crude yield) of a dark green solid. This material was recrystallized from carbon tetrachloride and vacuum filtered to give 8.4 grams (74%) of product as light green needles.

Sodium hydride (480 mg of a 50% mineral oil dispersion; 0.01 mol) was washed with reagent-grade hexane to remove the mineral oil and was suspended in 10 ml of freshly distilled THF ($N_2$ atmosphere and cooled to 0°C). 2-Chloro-4-trifluoromethyl-3′-(2-hydroxyethoxy)-4′-nitrodiphenyl ether (3,78 g, 0.01 mol) was added as a solution in 5 ml of THF. Hydrogen gas evolved slowly. Methyl dl 2-bromopropionate (1.67 g, 0.1 mol) was added dropwise (neat). A large subsequent evolution of gas was observed which subsided after 5 min. The ice bath was removed and the reaction was allowed to stir overnight at room temp.

Evaporation under reduced pressure gave a residue which was diluted with ether (50 ml) and 5% HCl (50 ml). After shaking in a separatory funnel the ether layer was retained. The aqueous phase was extracted a second time with ether (30 ml). The combined ether extracts were washed with aqueous $NaHCO_3$, brine, dried over anhydrous magnesium sulfate, filtered and concentrated to afford 3.34 g of a brown oil. Column chromatography using 200 g of 60—200 mesh silica gel eluted with 10% ethyl acetate/90% isooctane (1 liter), 15% ethyl acetate/85% isooctane (500 ml) and 20% ethyl acetate/80% isooctane, respectively, afforded 600 mg (13% yield) of a yellow oil which proved to be the desired product by NMR ($CDCl_3$) and elemental analysis.

*Method B:*

Sodium hydride (8.3 grams of a 50% dispersion in mineral oil; 0.173 mol) was washed with reagent-grade hexane to remove the mineral oil and was suspended in DMF (350 ml; dried over $CaH_2$). After cooling to 0°C, 2-chloro-4-trifluoromethyl-3′-hydroxy diphenyl ether (50 g, 0.173 mol) was added dropwise. Rapid evolution of gas was observed. After stirring 10—15 min., gas evolution ceased. 2-bromo-ethanol (64.83 g; 0.52 mol) was added to the cloudy grey suspension after which the reaction mixture was allowed to warm to room temp. and then was heated overnight at 60°C. After 24 hours another equivalent of 2-bromoethanol was added and the mixture was stirred and heated at ca. 75°C overnight. After cooling to room temp., Amberlite IRA—400—OH resin was added to remove starting material. The suspension was poured into water; an oil separated which was taken up into MDC (300 ml). The MDC extract was washed with water, dried over anhydrous magnesium sulfate, filtered and the filtrate evaporated under reduced pressure to afford 57.4 g (100%) of crude product.

Sodium hydride (6.02 g of a 50% dispersion in mineral oil; 0.14 mol) was weighed into a liter flask and washed with reagent-grade hexane to remove the mineral oil. Dimethylformamide (300 ml, dried over $CaH_2$) was added and the suspension was cooled to 0°C (ice bath). 2-Chloro-4-trifluoromethyl-3′-(2-hydroxyethoxy)-diphenyl ether (40 g., 0.12 mol) was added dropwise. When addition was complete, the

reaction was stirred 5 minutes at 0° and then stirred at room temperature until gas evolution stopped. Methyl dl 2-bromopropionate (24.1 g, 0.14 mol) was added rapidly. The reaction was exothermic with gas evolution; stirring was continued for 2½ hours then was heated at 60°C for 1 hour and finally stirred overnight at room temperature.

The reaction mixture was poured into cold water (500 ml), acidified (5% aqueous HCl) and extracted with MDC (2 × 150 ml). The combined MDC extracts were washed (5% NaOH, $H_2O$, saturated $NaHCO_3$, Brine), dried over anhydrous magnesium sulfate, filtered and the filtrate evaporated under reduced pressure to afford 46.6 g of a light golden oil. The crude material was separated by flash chromatography, using 12—13" of 60—200 mesh Davison Silica gel in a 40 mm i.d. column/10% ethyl acetate-90% isooctane solvent at a flow rate corresponding to a drop in the fluid level of 2"/min.

A 3-neck 250 ml round-bottomed flask equipped with a magnetic stirrer was charged with 8.18 g (0.02 mol) of 2-chloro-4-trifluoromethyl-3'-(1,4-dioxa-5-carbomethoxy-*n*-hexyl)-diphenyl ether, MDC (60 ml) and acetic anhydride (5.98 g; 0.059 mol). The solution was cooled to 0° (ice bath) and conc. $H_2SO_4$ (0.58 g; 0.0059 mol) was added dropwise. After 5 min. at 0°, 70% nitric acid (1.76 g; 0.02 mol) was added dropwise with vigorous stirring. The temperature was held at 0° for 1 hr. and was then allowed to warm to room temperature and stirred overnight.

The reaction mixture was poured into water/MDC (600 ml/200 ml) and the aqueous phase neutralized by the cautious addition of solid sodium bicarbonate to a pH of 7. The layers were separated and the organic phase washed ($H_2O$, saturated $NaHCO_3$, brine), dried over anhydrous magnesium sulfate, filtered and evaporated under reduced pressure to afford 8.31 g of a dark brown oil.

This procedure was repeated twice and the crude products combined to give 25 g of crude product which was purified by flash chromatography using 12—13" of 60—200 mesh Davison Silica gel in a 40 mm i.d. column/10% ethyl acetate-90% isooctane solvent at a flow rate corresponding to a drop in the fluid level of 2"/min. Thirty ml fractions were collected — fractions 75—110 were combined and evaporated under reduced pressure to give 8 g (29%) of pure product.

Example 3

Preparation of 2-chloro-4-trifluoromethyl-3'-(1,4-dioxa-5-carbomethoxy-n-hexyl)-4'-bromodiphenyl ether

Sodium hydride (0.24 g of a 50% suspension in mineral oil; 0.005 mol) was washed with reagent-grade hexane to remove the mineral oil and suspended in DMF (15 ml, dried over $CaH_2$). 2-Chloro-4-trifluoromethyl-3'-(2-hydroxyethoxy)-4'-bromodiphenyl ether (2.06 g; 0.005 mol) was added slowly with stirring. When gas evolution ceased, methyl dl 2-bromopropionate (1 g, 0.006 mol) was added and the reaction was stirred overnight at room temp ($N_2$ atm).

Because traces of starting alcohol were present (GL analysis) the reaction was heated at 70°C for 1 hr., allowed to cool and worked up by pouring the mixture into water (100 ml) and extraction with MDC (2 × 50 ml). The combined MDC extracts were washed successively with aqueous bicarbonate and brine, dried over anhydrous magnesium sulfate, filtered and concentrated under reduced pressure to afford 1.45 grams of a clear, colorless oil which by GC (3% OV—17) is a mixture of starting material and product. Separation was effected by wet column chromatography (MDC/silica gel). Starting material elutes first followed by 420 mg of Product (17% yield) as a clear colorless oil.

The compounds of Examples 5, 7, 8 and 17 were prepared in a similar manner. The above procedure was followed by ester exchange in preparing Example 17.

Example 9

Preparation of 2-chloro-4-trifluoromethyl-3'-(1,3-dioxa-4-carbomethoxy-n-pentyl)-4'-nitrodiphenyl ether

Methyl lactate (104 g) and paraformaldehyde (30 g) were placed in a suitable flask, stirred (magnetically) and cooled to 0—5°C (ice bath). Anhydrous hydrogen chloride was bubbled into the reaction mixture for 3½ hours. The paraformaldehyde slowly dissolved with separation of water. Upon completion, the organic layer was isolated and dried over a mixture of $MgSO_4$ and $K_2CO_3$. The liquid was then distilled to afford the desired compound (17.9 g), bp 45—60°C/0.1 mm which was used immediately after preparation. 2-Chloro-4-trifluoromethyl-3'-hydroxy-4'-nitrodiphenyl ether (3.34 g, 10 mmol) was completely dissolved in methylene chloride (25 ml). Anhydrous $K_2CO_3$ (1.52 g., 1.1 equiv.) was added in one portion. To the resulting slurry was added methyl 2-chloromethyloxypropionate with stirring. The reaction was stirred for 120 hours at room temperature, then filtered and stripped to a small volume. The residue was eluted through a short column of basic $Al_2O_3$ to yield a yellow oil (1.6 g, 36% yield).

Example 10

Preparation of 2-chloro-4-trifluoromethyl-3'-(1,4-dioxa-5-carbomethoxy-n-pentyl)-4'-nitrodiphenyl ether

2-Bromoethanol (125 g) and paraformaldehyde (30 g) were placed in a suitable flask, stirred (magnetically) and cooled to 0—5°C (ice bath). Anhydrous hydrogen chloride was bubbled into the reaction mixture for 3½ hours. The paraformaldehyde slowly dissolved with separation of water. Upon completion, the organic layer was isolated and dried over a mixture of $MgSO_60$ and $K_2CO_3$. The liquid was then distilled to afford the desired compound (125 g), bp 120°/23 mm which is stored at 0°C to prevent decomposition.

Copper (I) cyanide (28.6 g, 1.1 equivalents) was suspended in dry toluene (200 ml) with vigorous

stirring. 2-Bromoethyl chloromethylether (50.3 g) was added dropwise (*ca.* 5 min) and the resultant mixture was heated to a gentle reflux for 2½ hours. The solids were removed by filtration through Celite and the filtrate distilled to yield 38.1 g of product (bp 72—74°C/2.2 mm). The nitrile is either used immediately or stored at 0°C to prevent decomposition.

2-Bromoethoxyacetonitrile (38.1 g) was dissolved in reagent grade methanol at room temperature. The solution was maintained at 20—23°C (water bath). Anhydrous hydrogen chloride was bubbled into the solution for 2½ hours and the resultant mixture allowed to stand overnight. Heating over a steambath for 4 hours completed the reaction which was then cautiously diluted with water (500 ml). A liquid separated which was extracted into ether (3 × 150 ml). The combined extracts were dried over $MgSO_4$, filtered and evaporated under reduced pressure to yield 35.5 g of pure product. The material was used immediately after preparation.

2-Chloro-4-trifluoromethyl-3'-hydroxy-4'-nitrodiphenyl ether (33.4 g, 0.1 mol) was dissolved in dry dimethylformamide (200 ml). Anhydrous potassium carbonate (15.2 g, 0.11 FW) was added and the resulting stirred mixture was refluxed for 1 hr. The temperature was lowered to 100°C and methyl-2-(2-bromoethoxy)acetate (35.5 g) was added dropwise with stirring. The temperature was maintained at 100°C for 3 hr. after which the mixture was cooled to room temp. and poured into ice water (750 ml). A yellow oil separated which solidified upon standing to afford 44 g (98%) of very pure material, mp 84—85°.

The compounds of Examples 4, 5, 6, 7, 8, 11, 13, 14, 15, 16, 17 and 18 were prepared in a similar manner. The above procedure was followed by ester exchange in preparing Examples 4, 14 and 17 and ester hydrolysis in preparing Example 6.

Example 12

Preparation of 2-chloro-4-trifluoromethyl-3'-(1,4-dioxa-5-carboethoxy-n-hexyl)-4'-nitrodiphenyl ether

2-Chloro-4-trifluoromethyl-3'-(2-hydroxyethoxy)-4'-nitrodiphenyl ether (3.78 g, 10 mmol) was completely dissolved in methylene chloride (20 ml). Copper bronze (catalytic amt., 0.04 g) was added and the slurry was cooled to 0°C. Ethyl diazoacetate (0.14 g., 1 equiv.) was cautiously added dropwise with vigorous stirring. The reaction was allowed to come to room temperature and stirred for 18 hours. The reaction was quenched by pouring into water (50 ml) and the layers were separated. The organic layer was washed (5% HCl, brine), dried ($MgSO_4$), filtered and concentrated. The crude product was purified by chromatography using 20% EtOAc/80% isooctane as eluent to yield 0.93 g (20% of theoretical) of a yellow oil.

Following the procedures of Examples 1, 2, 3, 9, 10 and 12 and otherwise using conventional procedures well-known to those skilled in the art, other diphenyl ethers of Formula I as follows are prepared:

2-chloro-4-trifluoromethyl-3'-(1,4-dioxa-5-carboethoxy-*n*-hexyl)-4'-nitrodiphenyl ether
2-chloro-4-trifluoromethyl-3'-(1,4-dioxa-3-methyl-5-carbomethoxy-*n*-hexyl)-4'-nitrodiphenyl ether
2-chloro-4-trifluoromethyl-3'-(1,4-dioxa-5-carboxy-*n*-hexyl)-4'-nitrodiphenyl ether
2-chloro-4-trifluoromethyl-3'-(1,4-dioxa-3-methyl-5-carbomethoxy-*n*-pentyl)-4'-nitrodiphenyl ether
2-chloro-4-trifluoromethyl-3'-(1,4-dioxa-2-methyl-5-carbomethoxy-*n*-pentyl)-4'-nitrodiphenyl ether
2-chloro-4-trifluoromethyl-3'-(1,3-dioxa-4-carbomethoxy-*n*-pentyl)-4'-nitrodiphenyl ether
2-chloro-4-trifluoromethyl-3'-(1,4-dioxa-5-cyano-*n*-hexyl)-4'-nitrodiphenyl ether
2-chloro-4-trifluoromethyl-3'-(1,4-dioxa-5-carboethoxy-*n*-pentyl)-4'-nitrodiphenyl ether
2-chloro-4-trifluoromethyl-3'-(1,5-dioxa-6-carbomethoxy-*n*-hexyl)-4'-nitrodiphenyl ether
2-chloro-4-trifluoromethyl-3'-(1,4-dioxa-5-carboisopropoxy-*n*-pentyl)-4'-nitrodiphenyl ether
2-chloro-4-trifluoromethyl-3'-(1,5-dioxa-6-carbomethoxy-*n*-heptyl)-4'-nitrodiphenyl ether
2-chloro-4-trifluoromethyl-3'-(1,4-dioxa-5-carbomethoxy-*n*-heptyl)-4'-nitrodiphenyl ether
2-chloro-4-trifluoromethyl-3'-(1,4-dioxa-5-carboisopropoxy-*n*-hexyl)-4'-nitrodiphenyl ether
2-chloro-4-trifluoromethyl-3'-(1,4-dioxa-5-carbomethoxy-*n*-pentyl)-4'-bromodiphenyl ether
4-trifluoromethyl-3'-(1,4-dioza-5-carbomethoxy-*n*-pentyl)-4'-nitrodiphenyl ether
2-nitro-4-trifluoromethyl-3'-(1,4-dioxa-5-carbomethoxy-*n*-pentyl)-4'-nitrodiphenyl ether
2-cyano-4-trifluoromethyl-3'-(1,4-dioxa-5-carbomethoxy-*n*-pentyl)-4'-nitrodiphenyl ether
2,4-dichloro-3'-(1,4-dioxa-5-carbomethoxy-*n*-pentyl)-4'-nitrodiphenyl ether
2-chloro-4-cyano-3'-(1,4-dioxa-5-carbomethoxy-*n*-pentyl)-4'-nitrodiphenyl ether
2-chloro-4-trifluoromethyl-3'-(1,4-dioxa-5-carbomethoxy-*n*-pentyl)-4'-fluorodiphenyl ether
2-chloro-4-trifluoromethyl-3'-(1,4-dioxa-5-carbomethoxy-*n*-pentyl)-4'-cyanodiphenyl ether
2-chloro-4-trifluoromethyl-3'-(1,3-dioxa-4-carbomethoxy-*n*-butyl)-4'-nitrophenyl ether
2-chloro-4-trifluoromethyl-3'-(1,4-dioxa-2,3-dimethyl-5-carbomethoxy-*n*-pentyl)-4'-nitrodiphenyl ether
2-chloro-4-trifluoromethyl-3'-(1,4-dioxa-5-carbomethoxy-*n*-pentyl)-4'-methylthiodiphenyl ether
2-chloro-4-trifluoromethyl-3'-(1,4-dioza-5-carbomethoxy-*n*-pentyl)-4'-methylsulfinyl diphenyl ether
2-chloro-4-trifluoromethyl-3'-(1,4-dioxa-5-carbomethoxy-*n*-pentyl)-4'-methylsulfonyl diphenyl ether
2-chloro-4-trifluoromethyl-3'-(1,4-dioxa-5-carbomethoxy-*n*-pentyl)diphenyl ether
2-chloro-4-trifluoromethyl-3'-(1,4-dioxa-5-carboxamide-*n*-pentyl)-4'-nitrodiphenyl ether
2-chloro-4-trifluoromethyl-3'-(1,4-dioxa-5-carboxamide-*n*-hexyl)-4'-nitrodiphenyl ether
2-chloro-4-trifluoromethyl-3'-(1,4-dioxa-5-N,N-dimethyl-carboxamide-*n*-pentyl)-4'-nitrodiphenyl ether
2-chloro-4-trifluoromethyl-3'-(1,3-dioxa-2-methyl-4-carbomethoxy-*n*-butyl)-4'-nitrodiphenyl ether

The herbicidal activity of diphenyl ethers of the present invention towards a number of common weeds was evaluated. Using the procedures described below, the diphenyl ethers of the present invention were evaluated for control of the following weeds at 0.56 kg/ha (0.5 pounds per acre):

Monocots

| | |
|---|---|
| Barnyardgrass | (*Echinochloa crus-galli*) |
| Foxtail | (*Setaria viridis*) |
| Johnsongrass | (*Sorghum Halepense*) |
| Nutsedge | (*Cyperus esculentus*) |
| Wild Oat | (*Avena fatua*) |

Dicots

| | |
|---|---|
| Cocklebur | (*Xanthium pensylvanicum*) |
| Morningglory | (*Ipomoea spp.*) |
| Sicklepod | (*Cassia obtusifolia*) |
| Velvetleaf | (*Abutilon theophrasti*) |

The following test procedure was employed. Seeds of selected weeds are planted in soil in flats and covered with 1.3 cm (0.5 inch) of a 3:1 soil-sand mixture. For preemergence tests, immediately after planting, the test compound is sprayed directly onto the soil surface. The flats are then watered by subirrigation and overhead. Subsequently, only overhead watering is used. For postemergence tests, the seeds are allowed to germinate and after about two weeks the flats are treated with the test compound. The flats for postemergence tests are watered by subirrigation only.

The compound to be evaluated was dissolved in about 5.0 ml of 50:50 acetone-methanol solution with 0.05% (weight by volume) of an alkylaryl polyether alcohol surfactant (Triton X—114, commercially available from Rohm and Haas Co.). This solution is then diluted with about 20 ml of acetone and sprayed over the flats using a carrier volume equivalent to 76.5 l/ha (50 gallons per acre) at a 0.56 kg/ha (0.5 pounds per acre) rate of application. About two weeks after application of the test compound, the state of growth of the plant is observed. Table II gives average percent control achieved by the test compounds in terms of plants which were killed by the compounds.

| Compound of Example No. | | Herbicidal Activity (% Control) Preemergence | Postemergence |
|---|---|---|---|
| 1 | AD* | 10 | 51 |
| | AM* | 2 | 6 |
| 2 | AD | 86 | 100 |
| | AM | 55 | 71 |
| 3 | AD | 31 | 98 |
| | AM | 20 | 53 |
| 4 | AD | 66 | 90 |
| | AM | 28 | 54 |
| 5 | AD | 81 | 95 |
| | AM | 29 | 50 |
| 6 | AD | 73 | 100 |
| | AM | 31 | 39 |

TABLE II - (Cont'd)

| Compound of Example No. | | Herbicidal Activity (% Control) Preemergence | Postemergence |
|---|---|---|---|
| 7 | AD | 75 | 100 |
| | AM | 21 | 61 |
| 8 | AD | 81 | 100 |
| | AM | 29 | 51 |
| 9 | AD | 66 | 94 |
| | AM | 30 | 68 |
| 10 | AD | 100 | 100 |
| | AM | 25 | 60 |
| 11 | AD | 50 | 56 |
| | AM | 26 | 13 |
| 12 | AD | 79 | 100 |
| | AM | 26 | 56 |
| 13 | AD | 65 | 100 |
| | AM | 22 | 39 |
| 14 | AD | 75 | 100 |
| | AM | 24 | 50 |
| 15 | AD | 63 | 94 |
| | AM | 28 | 31 |
| 16 | AD | 46 | 100 |
| | AM | 26 | 55 |
| 17 | AD | 100 | 100 |
| | AM | 58 | 54 |
| 18 | AD | 69 | 88 |
| | AM | 6 | 52 |
| 19 | AD | 75 | 100 |
| | AM | 43 | 39 |
| 20 | AD | 75 | 100 |
| | AM | 24 | 22 |

*AD = Average of all dicots; AM = average of all monocots

**Claims for the Contracting States: BE CH DE FR GB IT LI LU NL SE**

1. A compound of the formula

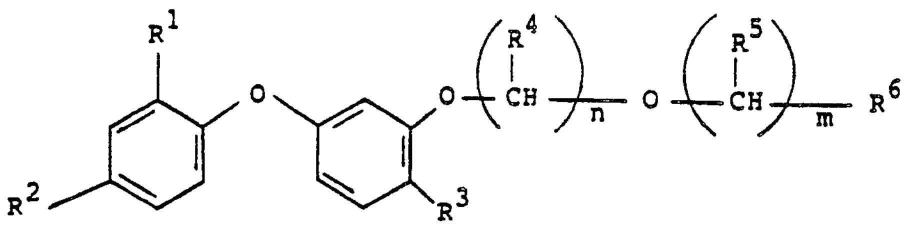

(I)

wherein:

$R^1$ is hydrogen, halogen, nitro or cyano;

$R^2$ is halogen, trifluoromethyl or cyano;

$R^3$ is hydrogen, nitro, halogen, cyano, $(C_1-C_4)$-alkylthio, $(C_1-C_4)$-alkylsulfinyl, or $(C_1-C_4)$-alkylsulfonyl;

$R^4$ is hydrogen or $(C_1-C_4)$ straight or branched chain alkyl;

$R^5$ is hydrogen or $(C_1-C_4)$ straight or branched chain alkyl;

$R^6$ is cyano or carboxy or an agronomically acceptable salt, ester or amide thereof;

n is an integer of one to four; and

m is an integer of one to four.

2. A compound according to claim 1 wherein:

$R^1$ is halogen;

$R^2$ is halogen or trifluoromethyl;

$R^3$ is nitro or halogen;

$R^4$ is hydrogen, methyl or ethyl;

$R^5$ is hydrogen, methyl or ethyl;

$R^6$ is carboxy or an agronomically acceptable salt, ester or amide thereof;

n is an integer of one or two; and

m is an integer of one or two.

3. A compound according to claim 2 wherein:

$R^1$ is chlorine;

$R^2$ is trifluoromethyl;

$R^3$ is nitro or halogen;

$R^4$ is hydrogen or methyl;

$R^5$ is hydrogen or methyl; and

$R^6$ is carboxy or an agronomically acceptable salt, ester or amide thereof.

4. A compound according to any preceding claim wherein $R^6$ is a carbalkoxy group ($CO_2R^7$) wherein $R^7$ is a $(C_1-C_3)$ straight or branched chain alkyl or a carboxyamide group ($CONR^8R^9$) where $R^8$ and $R^9$ are independently hydrogen, $(C_1-C_4)$alkyl or $(C_1-C_4)$alkoxy.

5. A compound according to claim 3 wherein

$R^3$ is bromine;

$R^4$ is hydrogen; and

$R^6$ is a carbalkoxy group ($CO_2R^7$) where $R^7$ is a $(C_1-C_3)$ straight or branched chain alkyl.

6. A herbicidal composition which comprises a herbicidally effective amount of a compound according to any preceding claim and an agronomically acceptable carrier or diluent, optionally comprising a surfactant.

7. A method of controlling weeds which comprises applying to the locus to be controlled a herbicidally effective amount of a compound according to any of claims 1 to 5 or a composition according to claim 6.

8. A method of claim 7 wherein the compound is applied at a rate of about 0.01 to 2.24 kg/ha (0.01 to 2 pounds per acre).

9. A process for producing a compound according to claim 1 comprising the steps of:

(1) reacting a diphenyl ether phenol of the formula

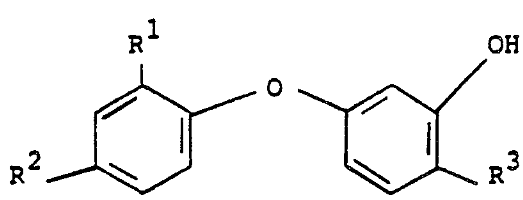

(II)

with substantially equimolar quantities of an alpha-haloaliphatic ether of the formula

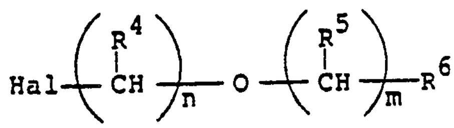

(III)

in the presence of an acid acceptor at a temperature of about 85 to 150°C for 1 to 24 hours to obtain a dioxaalkane carboxylic acid, ester, amide or salt of the formula

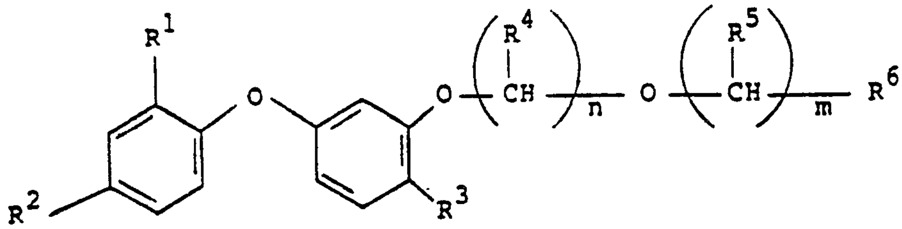

(I)

which can be isolated or hydrolyzed to the free acid and converted to obtain other esters, amides or salts; or

(2a) reacting a diphenyl ether phenol of the formula

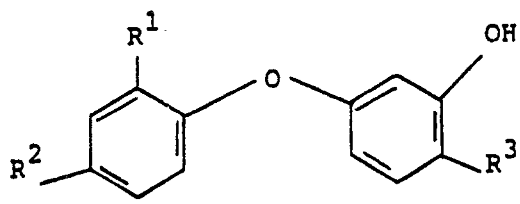

(II)

with substantially equimolar quantities of an alpha-haloaliphatic alcohol of the formula

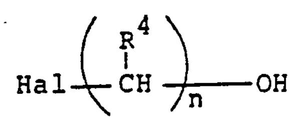

in the presence of an acid acceptor at a temperature of about 85 to 150°C for 1 to 24 hours to obtain a meta-substituted alcohol diphenyl ether intermediate of the formula

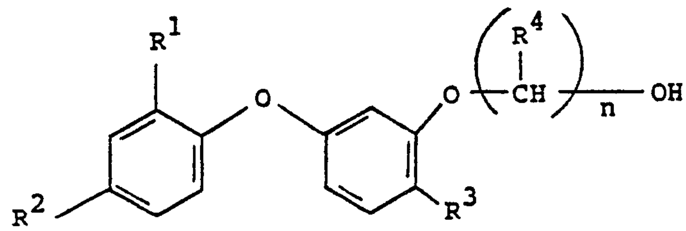

IV

or

(2b) reacting a diphenyl ether phenol of the formula

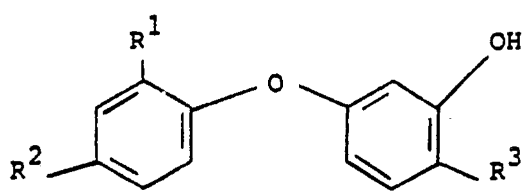

(II)

with substantially equimolar quantities of an alpha-halocarbonyl derivative of the formula

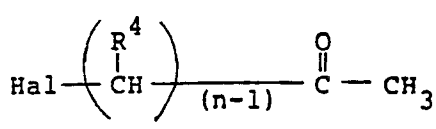

in the presence of an acid acceptor at a temperature of about 85 to 150°C for 1 to 24 hours to obtain an intermediate meta-substituted carbonyl diphenyl ether intermediate of the formula

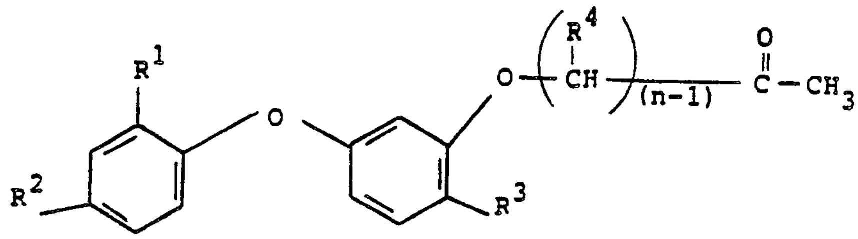

and

(3) reacting the intermediate from step (2b) with a reducing agent to obtain a meta-substituted alcohol diphenyl ether intermediate of the formula

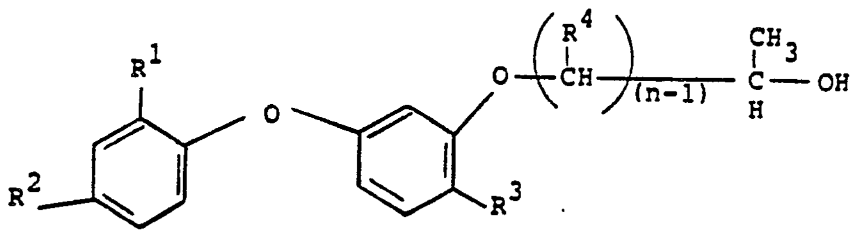

(IVa)

and

(4a) reacting intermediate from step (2a) or (3) with substantially equimolar quantities of an alpha-haloaliphatic of the formula

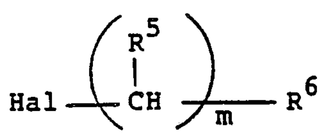

V

in the presence of an acid acceptor at a temperature of about −30°C to 5°C for 30 minutes to 20 hours or

(4b) reacting the intermediate from step (2a) or (3) with substantially equimolar quantities of an alkyl diazo of the formula

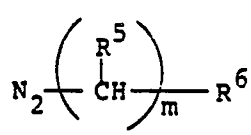

optionally in the presence of a catalyst at a temperature of about −30°C to 5°C for 30 minutes to 20 hours to obtain a dioxa-alkane carboxylic acid, ester, amide or salt of the formula

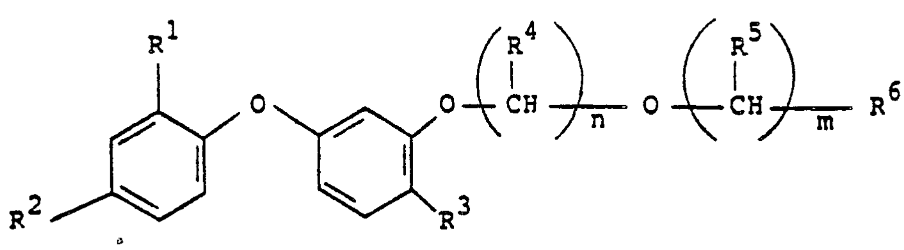

which can be isolated or hydrolyzed to the free acid and converted to obtain other esters, amides or salts; wherein $R^1$, $R^2$, $R^3$, $R^4$, $R^5$, $R^6$, n and m are as defined in claim 1.

10. A process according to claim 9 wherein $R^1$, $R^2$, $R^3$, $R^4$, $R^5$, $R^6$, n and m are as defined according to any one of claims 2 to 5.

**Claims for the Contracting State: AT**

1. An herbicidal composition comprising a compound of the formula

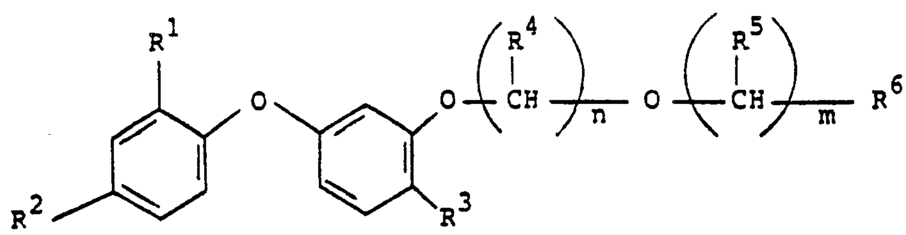

(I)

wherein:

$R^1$ is hydrogen, halogen, nitro or cyano;

$R^2$ is halogen, trifluoromethyl or cyano;

$R^3$ is hydrogen, nitro, halogen, cyano, $(C_1—C_4)$-alkylthio, $(C_1—C_4)$-alkylsulfinyl, or $(C_1—C_4)$-alkylsulfonyl;

$R^4$ is hydrogen or $(C_1—C_4)$ straight or branched chain alkyl;

$R^5$ is hydrogen or $(C_1—C_4)$ straight or branched chain alkyl;

$R^6$ is cyano or carboxy or an agronomically acceptable salt, ester or amide thereof;

n is an integer of one to four; and

m is an integer of one to four;

and an agronomically acceptable diluent or carrier.

2. A composition according to claim 1 wherein:

$R^1$ is halogen;

$R^2$ is halogen or trifluoromethyl;

$R^3$ is nitro or halogen;

$R^4$ is hydrogen, methyl or ethyl;

$R^5$ is hydrogen, methyl or ethyl;

$R^6$ is carboxy or an agronomically acceptable salt, ester or amide thereof;

n is an integer of one or two; and

m is an integer of one or two.

3. A composition according to claim 2 wherein:

$R^1$ is chlorine;

$R^2$ is trifluoromethyl;

$R^3$ is nitro or halogen;

$R^4$ is hydrogen or methyl;

$R^5$ is hydrogen or methyl; and

$R^6$ is carboxy or an agronomically acceptable salt, ester or amide thereof.

4. A composition according to any preceding claim wherein $R^6$ is a carbalkoxy group ($CO_2R^7$) wherein $R^7$ is a $(C_1—C_3)$ straight or branched chain alkyl or a carboxyamide group ($CONR^8R^9$) where $R^8$ and $R^9$ are independently hydrogen, $(C_1—C_4)$alkyl or $(C_1—C_4)$alkoxy.

5. A compound according to claim 3 wherein

$R^3$ is bromine;

$R^4$ is hydrogen; and

$R^6$ is a carbalkoxy group ($CO_2R^7$) where $R^7$ is a $(C_1—C_3)$ straight or branched chain alkyl.

6. A composition according to any preceding claim also comprising a surfactant.

7. A method of controlling weeds which comprises applying to the locus to be controlled a herbicidally effective amount of a compound of formula I as defined in any of claims 1 to 5 or a composition according to any of claims 1 to claim 6.

8. A method of claim 7 wherein the compound is applied at a rate of about 0.01 to 2.24 kg/ha (0.01 to 2 pounds per acre).

9. A process for producing a compound of formula I as defined in claim 1 comprising the steps of:

(1) reacting a diphenyl ether phenol of the formula

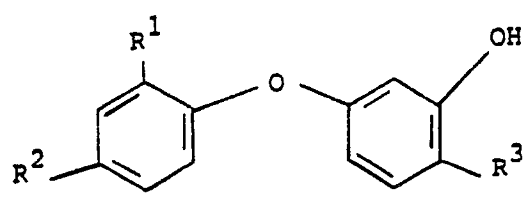

(II)

with substantially equimolar quantities of an alpha-haloaliphatic ether of the formula

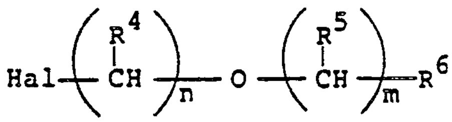

(III)

in the presence of an acid acceptor at a temperature of about 85 to 150°C for 1 to 24 hours to obtain a dioxaalkane carboxylic acid, ester, amide or salt of the formula

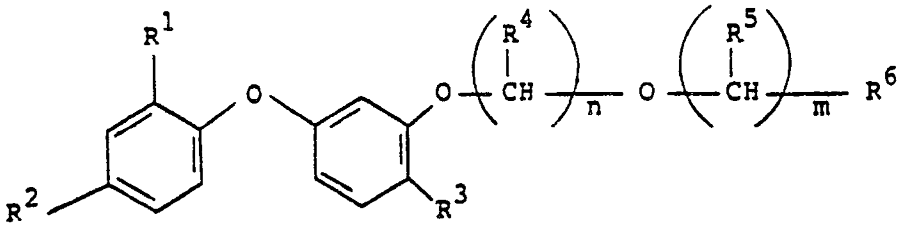

(I)

which can be isolated or hydrolyzed to the free acid and converted to obtain other esters, amides or salts; or

(2a) reacting a diphenyl ether phenol of the formula

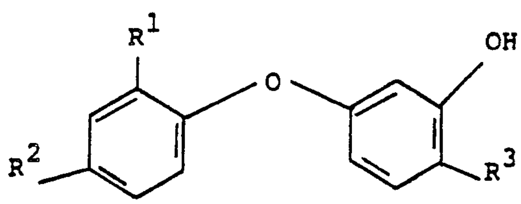

(II)

with substantially equimolar quantities of an alpha-haloaliphatic alcohol of the formula

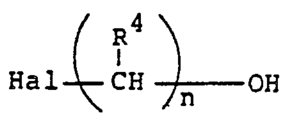

in the presence of an acid acceptor at a temperature of about 85 to 150°C for 1 to 24 hours to obtain a meta-substituted alcohol diphenyl ether intermediate of the formula

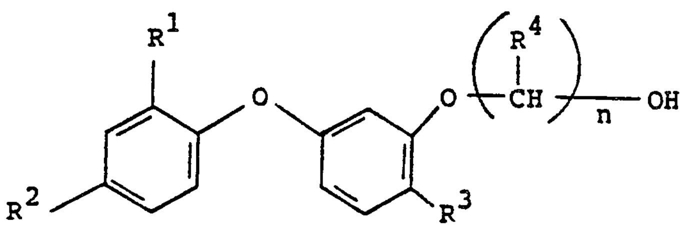

IV

or

(2b) reacting a diphenyl ether phenol of the formula

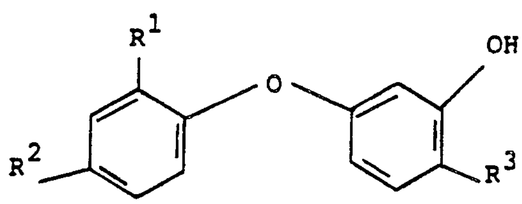

(II)

with substantially equimolar quantites of an alpha-halocarbonyl derivative of the formula

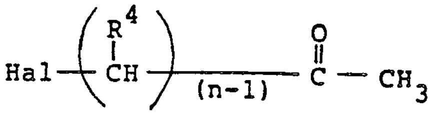

in the presence of an acid acceptor at a temperature of about 85 to 150°C for 1 to 24 hours to obtain an intermediate meta-substituted carbonyl diphenyl ether intermediate of the formula

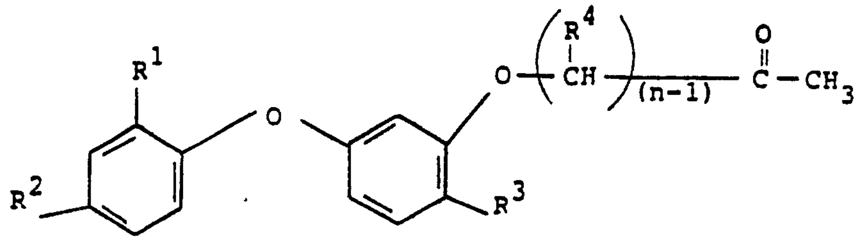

and

(3) reacting the intermediate from step (2b) with a reducing agent to obtain a meta-substituted alcohol diphenyl ether intermediate of the formula

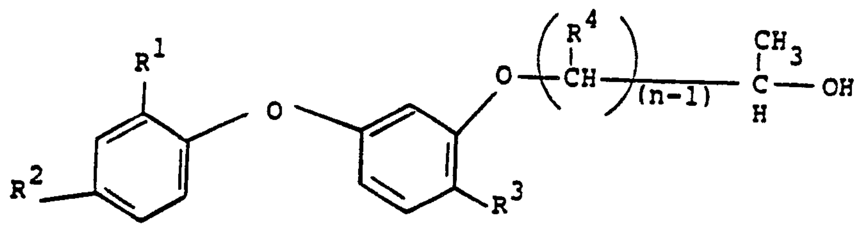

(IVa)

and

(4a) reacting intermediate from step (2a) or (3) with substantially equimolar quantities of an alpha-haloaliphatic of the formula

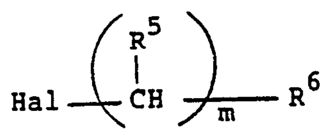

V

in the presence of an acid acceptor at a temperature of about −30°C to 5°C for 30 minutes to 20 hours or

(4b) reacting the intermediate from step (2a) or (3) with substantially equimolar quantities of an alkyl diazo of the formula

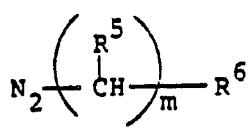

optionally in the presence of a catalyst at a temperature of about −30°C to 5°C for 30 minutes to 20 hours to obtain a dioxa-alkane carboxylic acid, ester, amide or salt of the formula

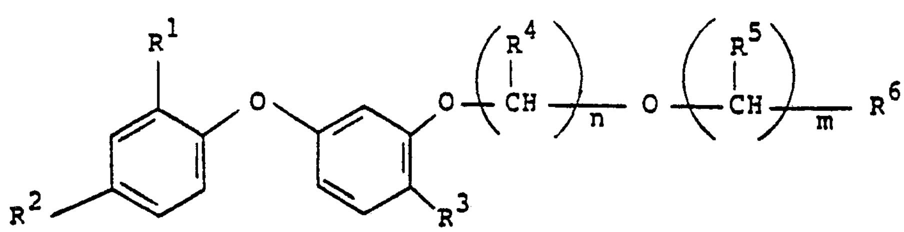

which can be isolated or hydrolyzed to the free acid and converted to obtain other esters, amides or salts; wherein $R^1$, $R^2$, $R^3$, $R^4$, $R^5$, $R^6$, n and m are as defined in claim 1.

10. A process according to claim 9 wherein $R^1$, $R^2$, $R^3$, $R^4$, $R^5$, $R^6$, n and m are each as defined in any one of claims 2 to 5.

**Patentansprüche für die Vertragsstaaten: BE CH DE FR GB IT LI LU NL SE**

1. Verbindung der Formel

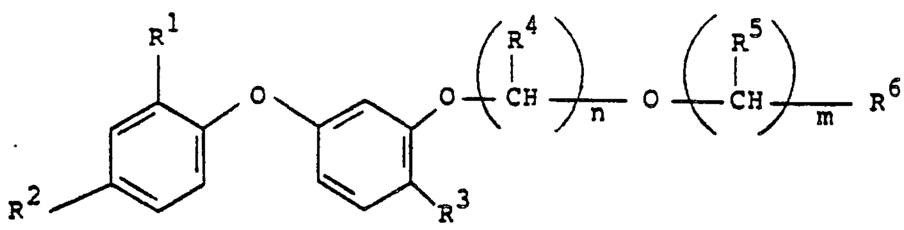

(I)

worin

$R^1$ Wasserstoff, Halogen, Nitro oder Cyano ist,

$R^2$ Halogen, Trifluormethyl oder Cyano ist,

$R^3$ Wasserstoff, Nitro, Halogen, Cyano, $(C_1—C_4)$Alkylthio, $(C_1—C_4)$Alkylsulfinyl oder $(C_1—C_4)$Alkylsulfonyl ist,

$R^4$ Wasserstoff oder geradkettiges oder verzweigtes $(C_1—C_4)$Alkyl ist,

$R^5$ Wasserstoff oder geradkettiges oder verzweigtes $(C_1—C_4)$Alkyl ist,

$R^6$ Cyano oder Carboxy oder ein für landwirtschaftliche Zwecke verträgliches Salz, Ester oder Amid davon ist,

n eine ganze Zahl von 1 bis 4 ist und

m eine ganze Zahl von 1 bis 4 ist.

2. Verbindung nach Anspruch 1, worin

$R^1$ Halogen ist,

$R^2$ Halogen oder Trifluormethyl ist,

$R^3$ Nitro oder Halogen ist,

$R^4$ Wasserstoff, Methyl oder Ethyl ist,

$R^5$ Wasserstoff, Methyl oder Ethyl ist,

$R^6$ Carboxy oder ein für landwirtshaftliche Zwecke verträgliches Salz, Ester oder Amid davon ist,

n eine ganze Zahl von 1 oder 2 ist und

m eine ganze Zahl von 1 oder 2 ist.

3. Verbindung nach Anspruch 2, worin

$R^1$ Chlor ist,

$R^2$ Trifluormethyl ist,

$R^3$ Nitro oder Halogen ist,

$R^4$ Wasserstoff oder Methyl ist,

$R^5$ Wasserstoff oder Methyl ist und

$R^6$ Carboxy oder ein für landwirtschafliche Zwecke verträgliches Salz, Ester oder Amid davon ist.

4. Verbindung gemäß einem der vorhergehenden Ansprüche, worin $R^6$ eine Carbalkoxygruppe $(CO_2R^7)$ ist, wobei $R^7$ eine geradkettige oder verzweigte $(C_1—C_3)$Alkyl- oder Carboxamidgruppe $(CONR^8R^9)$ ist, wobei $R^8$ und $R^9$ unabhängig voneinander Wasserstoff, $(C_1—C_4)$Alkyl oder $(C_1—C_4)$Alkoxy sind.

5. Verbindung nach Anspruch 3, worin

$R^3$ Brom ist,

$R^4$ Wasserstoff ist und

$R^6$ eine Carbalkoxygruppe $(CO_2R^7)$ ist, wobei $R^7$ geradkettiges oder verzweigtes $(C_1—C_3)$Alkyl ist.

6. Herbizides Mittel aus einer herbizid wirksamen Menge einer Verbindung gemäß einem der vorhergehenden Ansprüche und einem für landwirtschaftliche Zwecke verträglichen Träger oder Verdünnungsmittel, gegebenenfalls ein grenzflächenaktives Mittel enthaltend.

7. Verfahren zur Bekämpfung von Unkräutern, welches darin besteht, auf die zu bekämpfende Stelle eine herbizid wirksame Menge einer Verbindung gemäß einem der Ansprüche 1 bis 5 oder ein Mittel gemäß Anspruch 6 aufzubringen.

8. Verfahren nach Anspruch 7, wobei die Verbindung in einer Menge von ungefähr 0,01 bis 2,24 kg/ha (0,01 bis 2 pounds pro acre) aufgebracht wird.

9. Verfahren zur Herstellung einer Verbindung der Formel I, wie definiert in Anspruch 1, bestehend aus den folgenden Stufen:

(1) Umsetzung eines Diphenyletherphenols der Formel

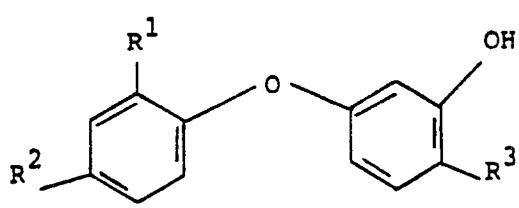

(II)

mit im wesentlichen äquimolaren Mengen eines alpha-halogenaliphatischen Ethers der Formel

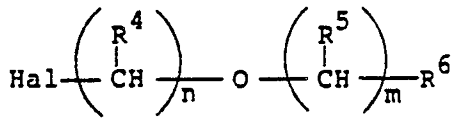

(III)

in Gegenwart eines Säureakzeptors bei einer Temperatur von ungefähr 85 bis 150°C während 1 bis 24 Stunden zur Gewinnung eines Dioxaalkancarbonsäureesters, Amides oder Salzes der Formel

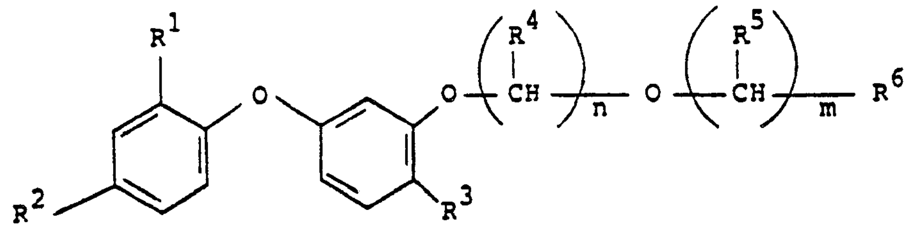

(I)

das zu der freien Säure isoliert oder hydrolysiert und zu Gewinnung von anderen Estern, Amiden oder Salzen umgewandelt werden kann, oder

(2a) Umsetzen eines Diphenyletherphenols der Formel

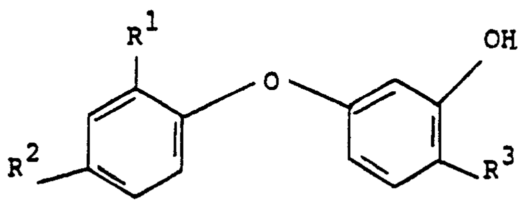

(II)

mit im wesentlichen äquimolaren Mengen eines alpha-halogenaliphatischen Alkohols der Formel

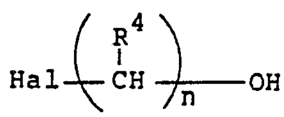

in Gegenwart eines Säureakzeptors bei einer Temperatur von ungefähr 85 bis 150°C während 1 bis 24 Stunden zur Gewinnung eines meta-substituierten Alkoholdiphenylether-Zwischenprodukts der Formel

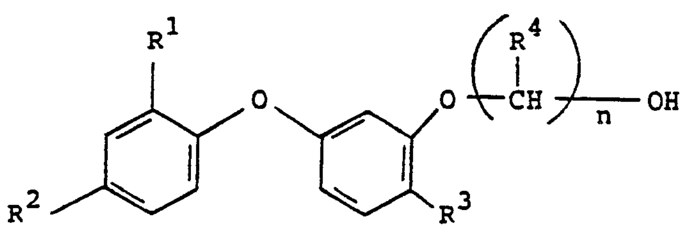

IV

oder

(2b) Umsetzen eines Diphenyletherphenols der Formel

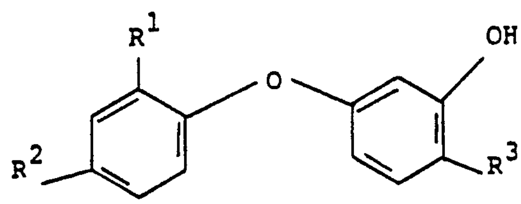

(II)

mit im wesentlichen äquimolaren Mengen eines alpha-Halogencarbonylderivates der Formel

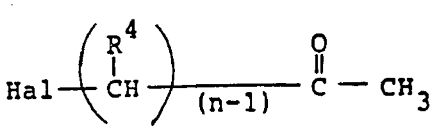

in Gegenwart eines Säureakzeptors bei einer Temperatur von ungefähr 85 bis 150°C während 1 bis 24 Stunden zur Gewinnung eines meta-substituierten Carbonyldiphenylether-Zwischenprodukts der Formel

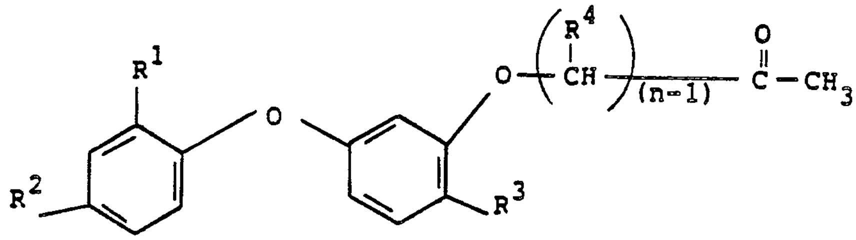

und

(3) Umsetzen des Zwischenprodukts aus der Stufe (2b) mit einem Reduktionsmittel zur Gewinnung eines meta-substitutierten Alkoholdiphenylether-Zwischenprodukts der Formel

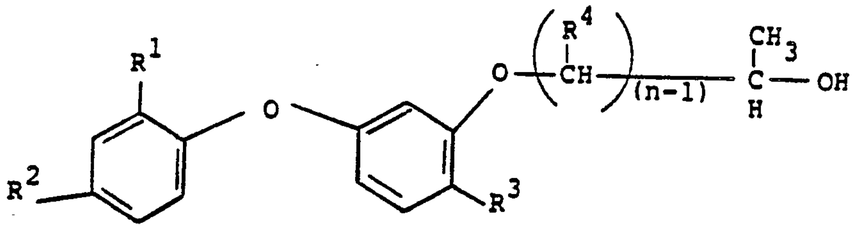

(IVa)

und

(4a) Umsetzen des Zwischenprodukts aus der Stufe (2a) oder (3) mit im wesentlichen äquimolaren Mengen einer alpha-halogenaliphatischen Verbindung der Formel

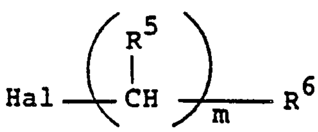

V

in Gegenwart eines Säureakzeptors bei einer Temperatur von ungefähr −30°C bis 5°C während 30 Minuten bis 20 Stunden oder

(4b) Umsetzen des Zwischenprodukts aus der Stufe (2a) oder (3) mit im wesentlichen äquimolaren Mengen einer Alkyldiazo-Verbindung der Formel

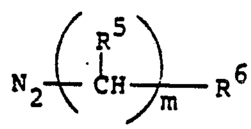

gegebenenfalls in Gegenwart eines Katalysators, bei einer Temperatur von ungefähr −30°C bis 5°C während 30 Minuten bis 20 Stunden zur Gewinnung einer Dioxaalkancarbonsäure, eines Esters, Amids oder Salzes der Formel

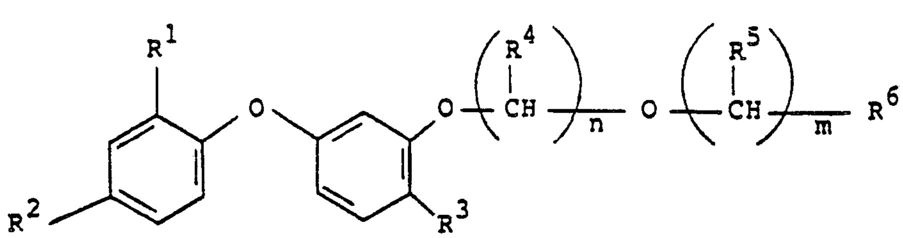

die bzw. das zu der frein Säure isoliert oder hydrolysiert und zur Gewinnung von anderen Estern, Amiden oder Salzen umgewandelt werden kann, worin $R^1$, $R^2$, $R^3$, $R^4$, $R^5$, $R^6$ n und m die in Anspruch 1 angegebenen Definitionen besitzen.

10. Verfahren nach Anspruch 9, wobei $R^1$, $R^2$, $R^3$, $R^4$, $R^5$, $R^6$ n und m jeweils einer der Definitionen der Ansprüche 2 bis 5 entsprechen.

**Patentansprüche für den Vertragsstaat: AT**

1. Herbizides Mittel, aufweisend eine Verbindng der Formel

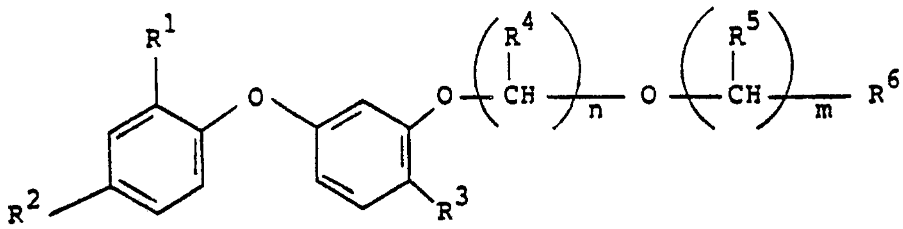

(I)

worin

$R^1$ Wasserstoff, Halogen, Nitro oder Cyano ist,

$R^2$ Halogen, Trifluormethyl oder Cyano ist,

$R^3$ Wasserstoff, Nitro, Halogen, Cyano, $(C_1-C_4)$Alkylthio, $(C_1-C_4)$Alkylsulfinyl oder $(C_1-C_4)$Alkylsulfonyl ist,

$R^4$ Wasserstoff oder geradkettiges oder verzweigtes $(C_1-C_4)$Alkyl ist,

$R^5$ Wasserstoff oder geradkettiges oder verzweigtes $(C_1-C_4)$Alkyl ist,

$R^6$ Cyano oder Carboxy oder ein für landwirtschaftliche Zwecke verträgliches Salz, Ester oder Amid davon ist,

n eine ganze Zahl von 1 bis 4 ist und

m eine ganze Zahl von 1 bis 4 ist

sowie ein für landwirtschaftliche Zwecke verträgliches Verdünnungsmittel oder ein für landwirtschaftliche Zwecke verträglichen Träger.

2. Mittel nach Anspruch 1, wobei

$R^1$ Halogen ist,

$R^2$ Halogen oder Trifluormethyl ist,

$R^3$ Nitro oder Halogen ist,

$R^4$ Wasserstoff, Methyl oder Ethyl ist,

$R^5$ Wasserstoff, Methyl oder Ethyl ist,

$R^6$ Carboxy oder ein für landwirtschaftliche Zwecke verträgliches Salz, Ester oder Amid davon ist,

n eine ganze Zahl von 1 oder 2 ist und

m eine ganze Zahl von 1 oder 2 ist.

3. Mittel nach Anspruch 2, wobei

$R^1$ Chlor ist,

$R^2$ Trifluormethyl ist,

$R^3$ Nitro oder Halogen ist,

$R^4$ Wasserstoff oder Methyl ist,

$R^5$ Wasserstoff oder Methyl ist und

$R^6$ Carboxy oder ein für landwirtschaftliche Zwecke verträgliches Salz, Ester oder Amid davon ist.

4. Mittel nach einem der vorhergehenden Ansprüche, wobei $R^6$ eine Carbalkoxygruppe $(CO_2R^7)$ ist, wobei $R^7$ eine geradkettige oder verzweigte $(C_1-C_3)$Alkyl- oder Carboxamidgruppe $(CONR^8R^9)$ ist, wobei $R^8$ und $R^9$ unabhängig voneinander Wasserstoff, $(C_1-C_4)$Alkyl oder $(C_1-C_4)$Alkoxy sind.

5. Mittel nach Anspruch 3, worin

$R^3$ Brom ist,

$R^4$ Wasserstoff ist und

$R^6$ eine Carbalkoxygruppe $(CO_2R^7)$ ist, wobei $R^7$ geradkettiges oder verzweigtes $(C_1-C_3)$Alkyl ist.

6. Mittel nach einem der vorhergehenden Ansprüche, das außerdem ein grenzflächenaktives Mittel enthält.

7. Verfahren zur Bekämpfung von Unkräutern, welches darin besteht, auf die zu bekämpfende Stelle eine herbizid wirksame Menge einer Verbindung der Formel I, wie definiert in einem der Ansprüche 1 bis 5, oder ein Mittel gemäß einem der Anspruch 1 bis 6 aufzubringen.

8. Verfahren nach Anspruch 7, wobei die Verbindung in einer Menge von ungefähr 0,01 bis 2,24 kg/ha (0,01 bis 2 pounds per acre) aufgebracht wird.

9. Verfahren zur Herstellung einer Verbindung der Formel I, wie definiert in Anspruch 1, bestehend aus den folgenden Stufen:

(1) Umsetzung eines Diphenyletherphenols der Formel

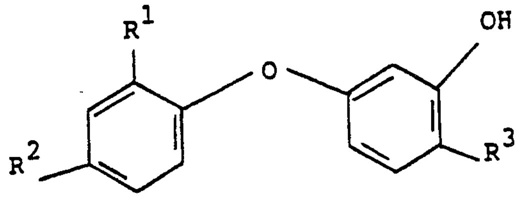

(II)

mit im wesentlichen äquimolaren Mengen eines alpha-halogenaliphatischen Ether der Formel

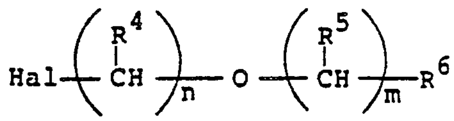

(III)

in Gegenwart eines Säureakzeptors bei einer Temperatur von ungefähr 85 bis 150°C während 1 bis 24 Stunden zur Gewinnung eines Dioxaalkancarbonsäureesters, Amides oder Salzes der Formel

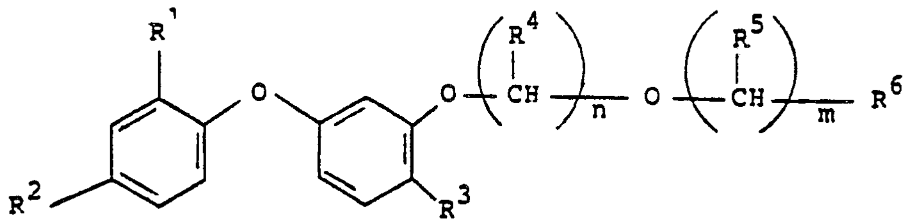

(I)

das zu der freien Säure isoliert oder hydrolysiert und zu Gewinnung von anderen Estern, Amiden oder Salzen umgewandelt werden kann, oder

(2a) Umsetzen eines Diphenyletherphenols der Formel

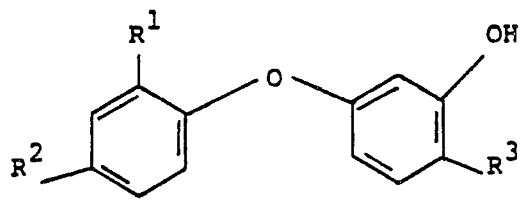

(II)

mit im wesentlichen äquimolaren Mengen eines alpha-halogenaliphatischen Alkohols der Formel

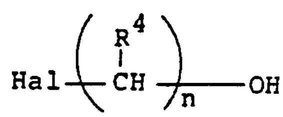

in Gegenwart eines Säureakzeptors bei einer Temperatur von ungefähr 85 bis 150°C während 1 bis 24 Stunden zur Gewinnung eines meta-substituierten Alkoholdiphenylether-Zwischenprodukts der Formel

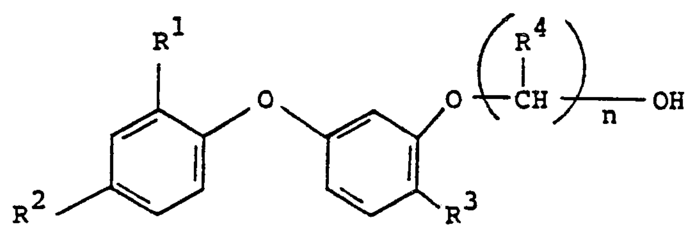

IV

oder

(2b) Umsetzen eines Diphenyletherphenols der Formel

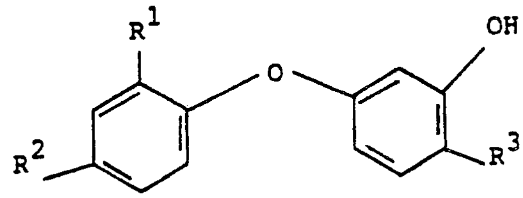

(II)

mit im wesentlichen äquimolaren Mengen eines alpha-Halogencarbonylderivates der Formel

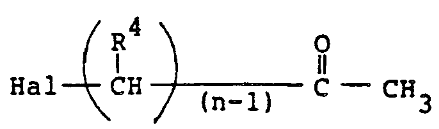

in Gegenwart eines Säureakzeptors bei einer Temperatur von ungefähr 85 bis 150°C während 1 bis 24 Stunden zur Gewinnung eines meta-substituierten Carbonyldiphenylether-Zwischenprodukts der Formel

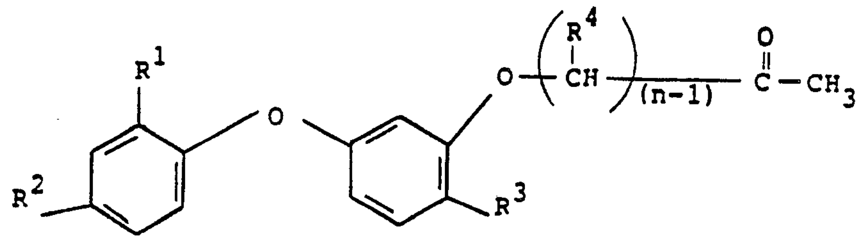

und

(3) Umsetzen des Zwischenprodukts aus der Stufe (2b) mit einem Reduktionsmittel zur Gewinnung eines meta-substituierten Alkoholdiphenylether-Zwischenprodukts der Formel

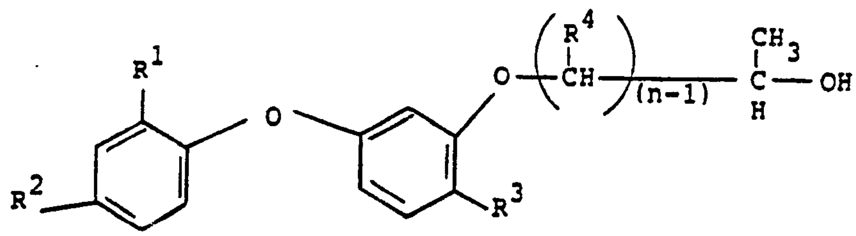

(IVa)

und

(4a) Umsetzen des Zwischenprodukts aus der Stufe (2a) oder (3) mit im wesentlichen äquimolaren Mengen einer alpha-halogenaliphatischen Verbindung der Formel

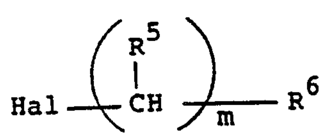

V

in Gegenwart eines Säureakzeptors bei einer Temperatur von ungefähr −30°C bis 5°C während 30 Minuten bis zwei Stunden oder

(4b) Umsetzen des Zwischenprodukts aus der Stufe (2a) oder (3) mit im wesentlichen äquimolaren Mengen einer Alkyldiazo-Verbindung der Formel

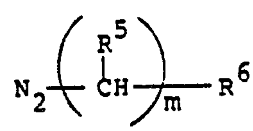

gegebenenfalls in Gegenwart eines Katalysators, bei einer Temperatur von ungefähr −30°C bis 5°C während 30 Minuten bis 20 Stunden zur Gewinnung einer Dioxaalkancarbonsäure, eines -esters, -amids oder -salzes der Formel

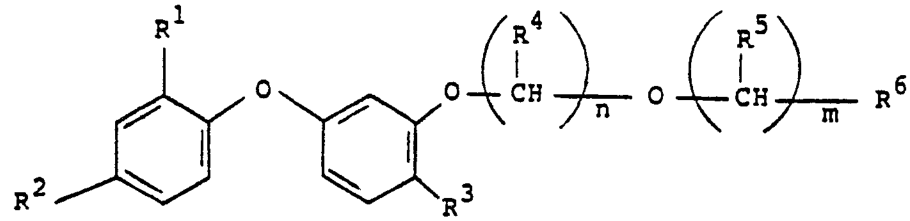

die bzw. das in der freien Säure isoliert oder hydrolysiert und zur Gewinnung von anderen Estern, Amiden oder Salzen umgewandelt werden kann, worin $R^1$, $R^2$, $R^3$, $R^4$, $R^5$, $R^6$ n und m die in Anspruch 1 angegebenen Definitionen besitzen.

10. Verfahren nach Anspruch 9, wobei $R^1$, $R^2$, $R^3$, $R^4$, $R^5$, $R^6$ n und m jeweils einer der Definitionen der Ansprüche 2 bis 5 entsprechen.

**Revendications pour les Etats contractants: BE CH DE FR GB IT LI LU NL SE**

1. Un composé de formule

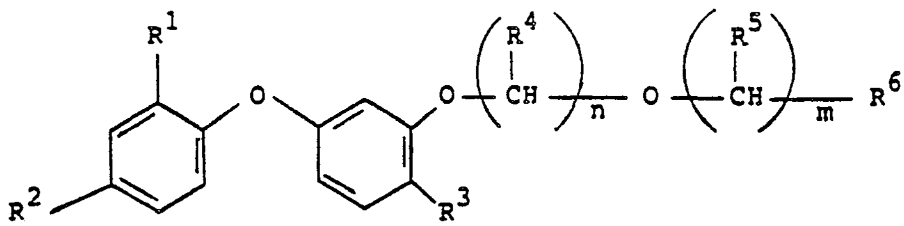

(I)

dans laquelle:

$R^1$ est un hydrogène, un halogène, un nitro ou un cyano;

$R^2$ est un halogène, un trifluorométhyle ou un cyano;

$R^3$ est un hydrogène, un nitro, un halogène, un cyano, un alcoylthio en $C_1$—$C_4$, un alcoylsulfinyle en $C_1$—$C_4$ ou un alcoylsulfonyle en $C_1$—$C_4$;

$R^4$ est un hydrogène ou un alcoyle en $C_1$—$C_4$ à chaîne droite ou ramifiée;

$R^5$ est un hydrogène ou un alcoyle en $C_1$—$C_4$ à chaîne droite ou ramifiée;

$R^6$ est un cyano ou un carboxy ou un sel, ester ou amide convenant en agronomie de celui-ci;

n est un entier de un à quatre; et

m est un entier de un à quatre.

2. Un composé selon la revendication 1, où:

$R^1$ est un halogène;

$R^2$ est un halogène ou un trifluorométhyle;

$R^3$ est un nitro ou un halogène;

$R^4$ est un hydrogène, un méthyle ou un éthyle;

$R^5$ est un hydrogène, un méthyle ou un éthyle;

$R^6$ est un carboxy ou un sel, ester ou amide convenant en agronomie de celui-ci;

n est un entier valant un ou deux; et

m est un entier valant un ou deux.

3. Un composé selon la revendication 2, où:

$R^1$ est un chlore;

$R^2$ est un trifluorométhyle;

$R^3$ est un nitro ou un halogène;

$R^4$ est un hydrogène ou un méthyle;

$R^5$ est un hydrogène ou un méthyle; et

$R^6$ est un carboxy ou un sel, ester ou amide convenant en agronomie de celui-ci.

4. Un composé selon l'une quelconque des revendications précédentes, où $R^6$ est un groupe carbalcoxy ($CO_2R^7$) où $R^7$ est un alcoyle en $C_1$—$C_3$ à chaîne droite ou ramifiée ou un groupe carboxamide ($CONR^8R^9$) où $R^8$ et $R^9$ sont indépendamment un hydrogène, un alcoyle en $C_1$—$C_4$ ou un alcoxy en $C_1$—$C_4$.

5. Un composé selon la revendication 3, où

$R^3$ est un brome;

$R^4$ est un hydrogène; et

$R^6$ est un groupe carbalcoxy ($CO_2R^7$) où $R^7$ est un alcoyle en $C_1$—$C_3$ à chaîne droite ou ramifiée.

6. Une composition herbicide qui comprend une quantité herbicide efficace d'un composé selon l'une quelconque des revendications précédentes et un véhicule ou diluant convenant en agronomie, comprenant facultativement un agent tensio-actif.

7. Un procédé de maîtrise des mauvaises herbes qui comprend l'application au site à maîtriser d'une quantité herbicide efficace d'un composé selon l'une quelconque des revendications 1 à 5 ou d'une composition selon la revendication 6.

8. Un procédé selon la revendication 7, dans lequel le composé est appliqué à la dose d'environ 0,01 à 2,24 kg/ha (0,01 à 2 pounds par acre).

9. Un procédé pour produire un composé selon la revendication 1 comprenant les stades de:

(1) réaction d'un éther diphénylique-phénol de formule

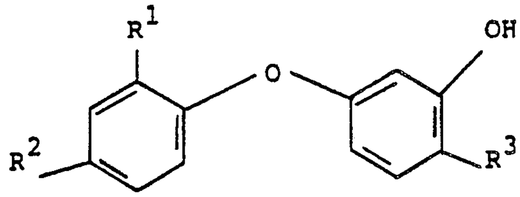

(II)

avec des quantités sensiblement équimolaires d'un éther α-halogénoaliphatique de formule

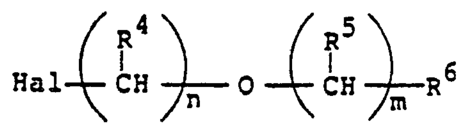

(III)

en présence d'un accepteur d'acide à une température d'environ 85 à 150°C pendant 1 à 24 heures, pour obtenir un ester, amide ou sel dioxaalcanecarboxylique de formule

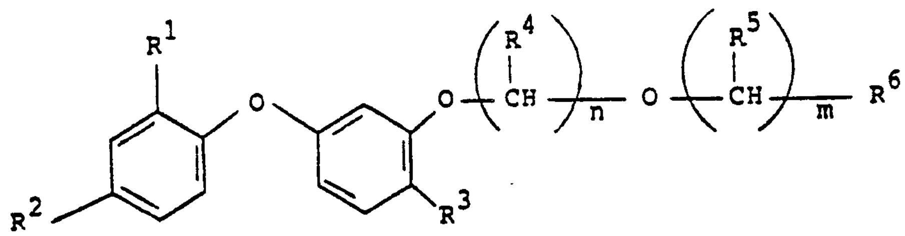

(I)

que l'on peut isoler ou hydrolyser en l'acide libre et transformer pour obtenir d'autres esters, amides ou sels; ou

(2a) réaction d'un éther diphénylique-phénol de formule

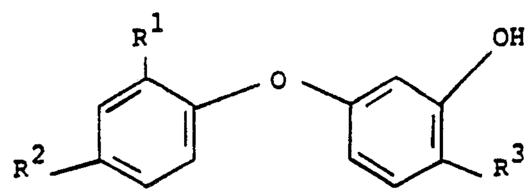

(II)

avec des quantités sensiblement équimolaires d'un alcool α-halogénoaliphatique de formule

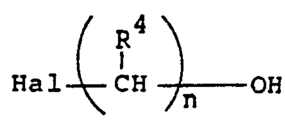

en présence d'un accepteur d'acide à une température d'environ 85 à 150°C pendant 1 à 24 heures, pour obtenir un éther diphénylique-alcool méta-substitué intermédiaire de formule

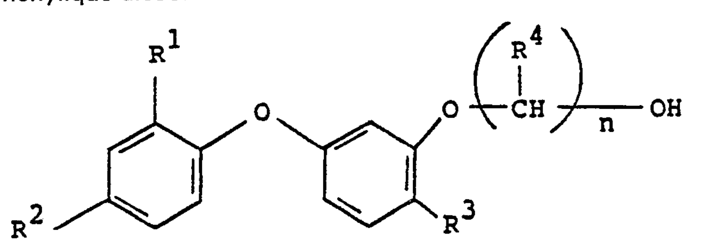

IV

ou

(2b) réaction d'un éther diphénylique-phénol de formule

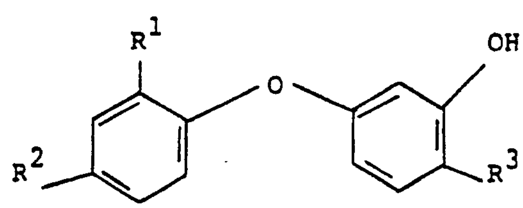

(II)

avec des quantitiés sensiblement équimolaires d'un dérivé α-halogénocarbonylique de formule

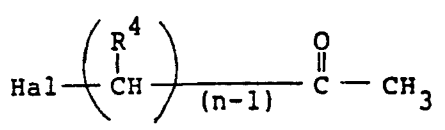

en présence d'un accepteur d'acide à une température d'environ 85 à 150°C pendant 1 à 24 heures, pour obtenir un éther diphénylique caronylé méta-substitué intermédiaire de formule

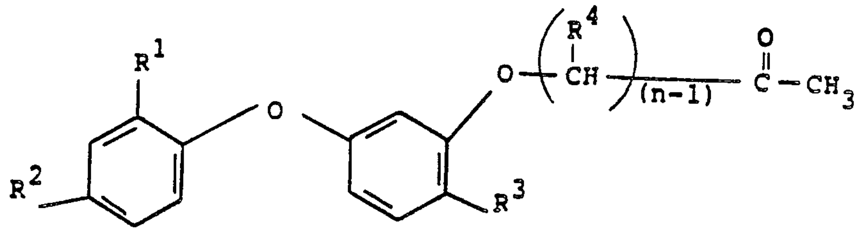

et

(3) réaction de l'intermédiaire du stade (2b) avec un agent réducteur pour obtenir un éther diphénylique-alcool méta-substitué intermédiaire de formule

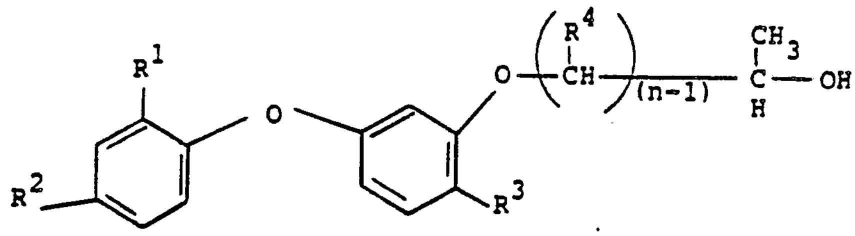

(IVa)

et

(4a) réaction de l'intermédiaire du stade (2a) ou (3) avec des quantités sensiblement équimolaires d'un composé α-halogénoaliphatique de formule

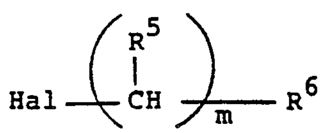

V

en présence d'un accepteur d'acide à une température d'environ −30°C à 5°C pendant 30 minutes à 20 heures

ou

(4b) réaction de l'intermédiaire du stade (2a) ou (3) avec des quantités sensiblement équimolaires d'un composé alcoyldiazoïque de formule

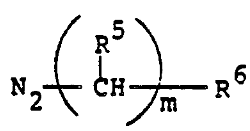

facultativement en présence d'un catalyseur à une température d'environ −30°C à 5°C pendant 30 minutes à 20 heures pour obtenir un acide, ester, amide ou sel dioxaalcanecarboxylique de formule

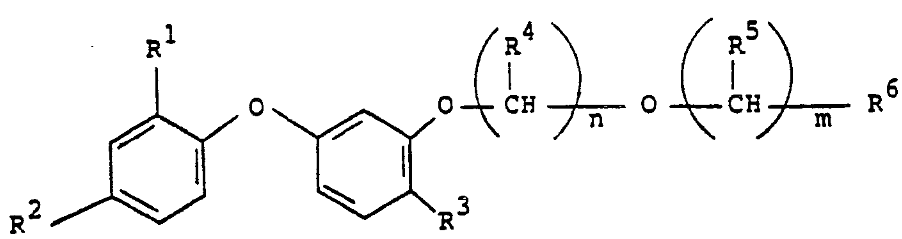

que l'on peut isoler ou hydrolyser en l'acide libre et transformer pour obtenir d'autres esters, amides ou sels; où $R^1$, $R^2$, $R^3$, $R^4$, $R^5$, $R^6$ n et m sont comme défini dans la revendication 1.

10. Un procédé selon la revendication 9, où $R^1$, $R^2$, $R^3$, $R^4$, $R^5$, $R^6$ n et m sont chacun comme défini dans l'une quelconque des revendications 2 à 5.

**Revendications pour l'Etat contractant: AT**

1. Une composition herbicide comprenant un composé de formule

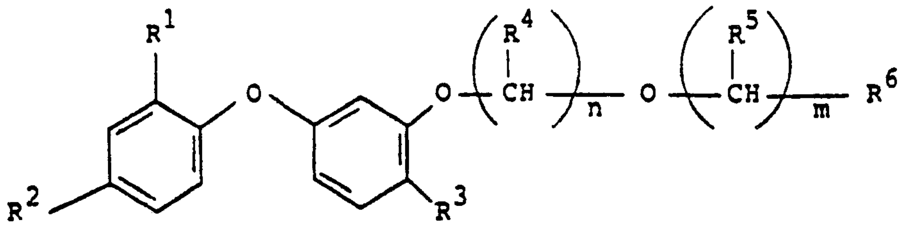

(I)

dans laquelle:

$R^1$ est un hydrogène, un halogène, un nitro ou un cyano;

$R^2$ est un halogène, un trifluorométhyle ou un cyano;

$R^3$ est un hydrogène, un nitro, un halogène, un cyano, un alcoylthio en $C_1$—$C_4$, un alcoylsulfinyle en $C_1$—$C_4$ ou un alcoylsulfonyle en $C_1$—$C_4$;

$R^4$ est un hydrogène ou un alcoyle en $C_1$—$C_4$ à chaîne droite ou ramifiée;

$R^5$ est un hydrogène ou un alcoyle en $C_1$—$C_4$ à chaîne droite ou ramifiée;

$R^6$ est un cyano ou un carboxy ou un sel, ester ou amide convenant en agronomie de celui-ci;

n est un entier de un à quatre; et

m est un entier de un à quatre;

et un diluant ou véhicule convenant en agronomie.

2. Une composition selon la revendication 1, où:

$R^1$ est un halogène;

$R^2$ est un halogène ou un trifluorométhyle;

$R^3$ est un nitro ou un halogène;

$R^4$ est un hydrogène, un méthyle ou un éthyle;

$R^5$ est un hydrogène, un méthyle ou un éthyle;

$R^6$ est un carboxy ou un sel, ester ou amide convenant en agronomie de celui-ci;

n est un entier valant un ou deux; et

m est un entier valant un ou deux.

3. Une composition selon la revendication 2, où:

$R^1$ est un chlore;

$R^2$ est un trifluorométhyle;

$R^3$ est un nitro ou un halogène;

$R^4$ est un hydrogène ou un méthyle;

$R^5$ est un hydrogène ou un méthyle; et

$R^6$ est un carboxy ou un sel, ester ou amide convenant en agronomie de celui-ci.

4. Une composition selon l'une quelconque des revendications précédentes, où $R^6$ est un groupe carbalcoxy ($CO_2R^7$) ou $R^7$ est un alcoyle en $C_1$—$C_3$ à chaîne droite ou ramifiée ou un groupe carboxamide ($CONR^8R^9$) où $R^8$ et $R^9$ sont indépendamment un hydrogène, un alcoyle en $C_1$—$C_4$ ou un alcoxy en $C_1$—$C_4$.

5. Une composition selon la revendication 3, où

$R^3$ est un brome;

$R^4$ est un hydrogène; et

$R^6$ est un groupe carbalcoxy ($CO_2R^7$) où $R^7$ est un alcoyle en $C_1$—$C_3$ à chaîne droite ou ramifiée.

6. Une composition selon l'une quelconque des revendications précédentes comprenant également un agent tensio-actif.

7. Un procédé de maîtrise des mauvaises herbes qui comprend l'application au site à maîtriser d'une quantité herbicide efficace d'un composé de formule I comme défini dans l'une quelconque des revendications 1 à 5 ou d'une composition selon l'une quelconque des revendications 1 à 6.

8. Un procédé selon la revendication 7, dans lequel le composé est appliqué à une dose d'environ 0,01 à 2,24 kg/ha (0,01 à 2 pounds par acre).

9. Un procédé pour produire un composé de formule I comme défini dans la revendication 1 comprenant les stades de:

(1) réaction d'un éther diphénylique-phénol de formule

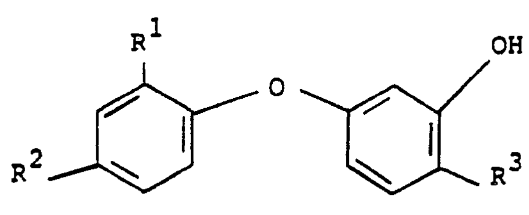

(II)

avec des quantités sensiblement équimolaires d'un éther α-halogénoaliphatique de formule

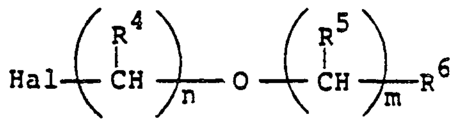

(III)

en présence d'un accepteur d'acide à une température d'environ 85 à 150°C pendant 1 à 24 heures, pour obtenir un ester, amide ou sel dioxaalcanecarboxylique de formule

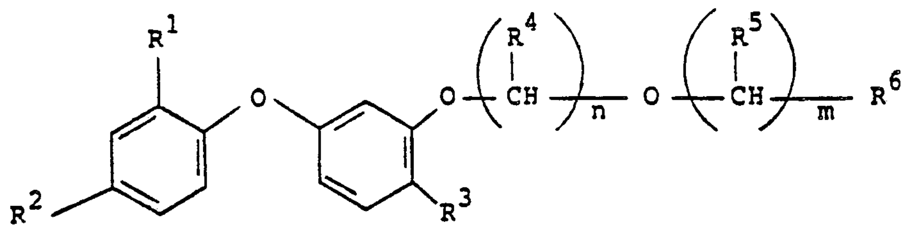

(I)

que l'on peut isoler ou hydrolyser en l'acide libre et transformer pour obtenir d'autres esters, amides ou sels; ou

(2a) réaction d'un éther diphénylique-phénol de formule

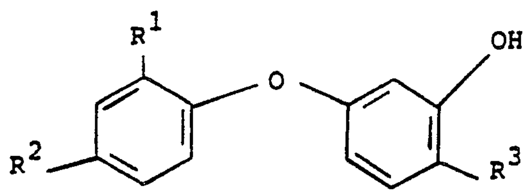

(II)

avec des quantités sensiblement équimolaires d'un alcool α-halogénoaliphatique de formule

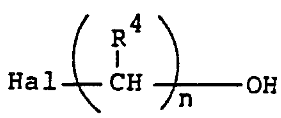

en présence d'un accepteur d'acide à une température d'environ 85 à 150°C pendant 1 à 24 heures, pour obtenir un éther diphénylique-alcool méta-substitué intermédiaire de formule

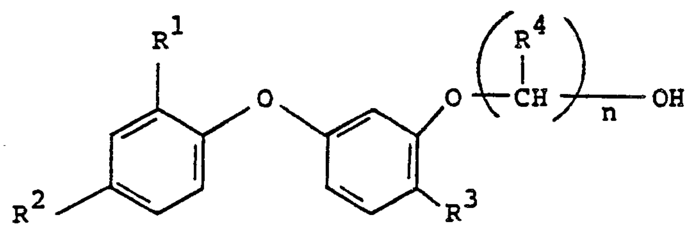

IV

ou

(2b) réaction d'un éther diphénylique-phénol de formule

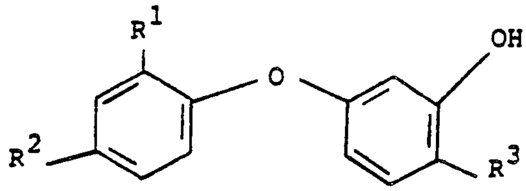

(II)

avec des quantités sensiblement équimolaires d'un dérivé α-halogénocarbonylique de formule

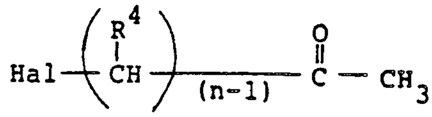

en présence d'un accepteur d'acide à une température d'environ 85 à 150°C pendant 1 à 24 heures, pour obtenir un éther diphénylique caronylé méta-substitué intermédiaire de formule

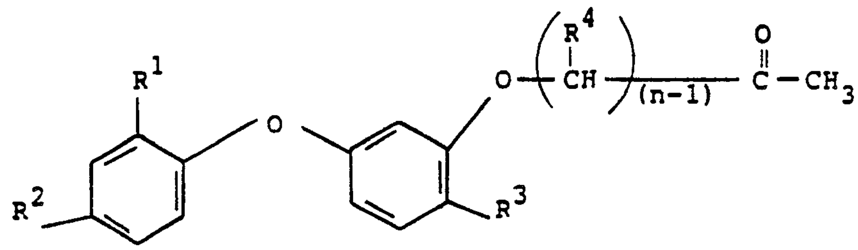

et

(3) réaction de l'intermédiaire du stade (2b) avec un agent réducteur pour obtenir un éther diphénylique-alcool méta-substitué intermédiaire de formule

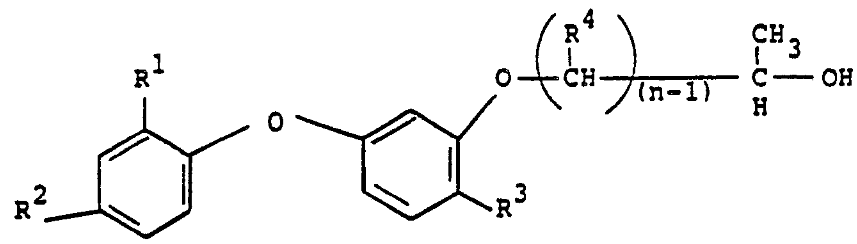

(IVa)

(4a) réaction de l'intermédiaire du stade (2a) ou (3) avec des quantités sensiblement équimolaires d'un composé α-halogénoaliphatique de formule

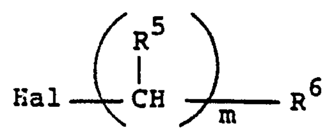

V

en présence d'un accepteur d'acide à une température d'environ −30°C à 5°C pendant 30 minutes à 20 heures

ou

(4b) réaction de l'intermédiaire du stade (2a) ou (3) avec des quantités sensiblement équimolaires d'un composé alcoyldiazoïque de formule

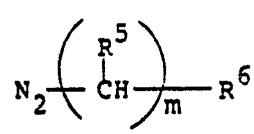

facultativement en présence d'un catalyseur à une température d'environ −30°C à 5°C pendant 30 minutes à 20 heures pour obtenir un acide, ester, amide ou sel dioxaalcanecarboxylique de formule

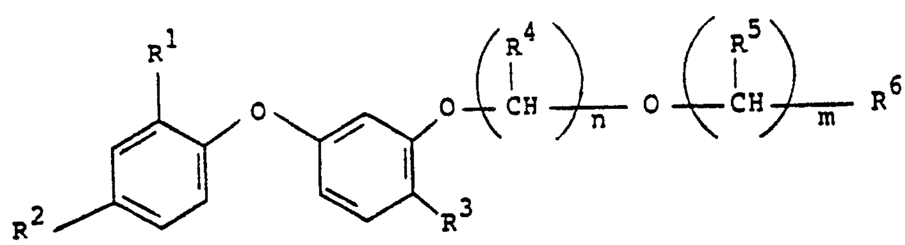

que l'on peut isoler ou hydrolyser en l'acide libre et transformer pour obtenir d'autres esters, amides ou sels; où $R^1$, $R^2$, $R^3$, $R^4$, $R^5$, $R^6$ n et m sont comme défini dans la revendication 1.

10. Un procédé selon la revendication 9, où $R^1$, $R^2$, $R^3$, $R^4$, $R^5$, $R^6$ n et m sont chacun comme défini dans l'une quelconque des revendications 2 à 5.